(19) Europäisches Patentamt / European Patent Office / Office européen des brevets

(11) **EP 4 733 268 A1**

(12) **DEMANDE DE BREVET EUROPEEN**

(43) Date de publication:
**29.04.2026 Bulletin 2026/18**

(21) Numéro de dépôt: **25210404.7**

(22) Date de dépôt: **22.10.2025**

(51) Classification Internationale des Brevets (IPC):
*C02F 1/00* $^{(2023.01)}$     *G01F 23/00* $^{(2022.01)}$
*G01F 23/24* $^{(2006.01)}$     *G01F 23/80* $^{(2022.01)}$
*G01N 27/06* $^{(2006.01)}$     *G01N 27/07* $^{(2006.01)}$
*G01N 27/08* $^{(2006.01)}$     *G01N 33/18* $^{(2006.01)}$

(52) Classification Coopérative des Brevets (CPC):
**G01N 27/07; G01F 23/243; G01F 23/804;
G01N 27/08; G01N 33/1826**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **24.10.2024 FR 2411647**

(71) Demandeur: **Ijinus
29300 Mellac (FR)**

(72) Inventeurs:
• **ZUG, Mathieu
44170 VAY (FR)**
• **LHOMME, Nicolas
73520 La Bridoire (FR)**

(74) Mandataire: **Jacobacci & Partners France
32, rue de l'Arcade
75008 Paris (FR)**

(54) **SYSTÈME ET PROCÉDÉ DE MESURE D'AU MOINS UNE CARACTÉRISTIQUE D'UNE EAU D'UN RÉSEAU D'ASSAINISSEMENT**

(57) Un procédé de mesure d'au moins un paramètre d'une eau d'un réseau d'assainissement est divulgué, dans lequel:

a) on met en œuvre un capteur (1) comportant des paires d'électrodes étagées, et,

b) dans une phase de mesures, on mesure pour chaque paire d'électrodes du capteur un courant électrique local 1 et un voltage local $V_i$, et

c) on corrige les courants électriques locaux en ôtant une valeur de décalage Z de mesure de courant pour produire des courants électriques locaux corrigés, et

d) on calcule des conductances locales $Co_i$ corrigées en utilisant les courants électriques locaux corrigés, et

e) on calcule des conductivités locales $EC_{25i}$ en utilisant les produits des conductances locales $Co_i$ par des constantes de cellule $K_i$ et en utilisant une mesure de température T de ladite eau pour corriger un effet de la température sur les conductances locales, et

f) on détermine une hauteur relative $Hr_{(mesure)}$ d'eau et

g) on calcule une conductivité globale $EC_{25}$ de ladite eau par une fonction ayant au moins un paramètre qui est la hauteur relative $Hr_{(mesure)}$ déterminée de ladite eau.

Le procédé est mis en œuvre dans un système.

[Fig. 7]

## Description

### Domaine de l'invention

L'invention concerne un système et procédé de mesure d'au moins une caractéristique d'une eau d'un réseau d'assainissement par calcul de la conductivité de ladite eau et d'un niveau (ou hauteur) de ladite eau afin de permettre, en particulier, la détection de la présence d'eaux parasites et possiblement de les quantifier, la détection de la présence de sédiments et possiblement de les quantifier, de calculer un débit de ladite eau. Elle a des applications dans le domaine de la surveillance des réseaux d'assainissement et la détection des eaux parasites.

### État de la technique

La présente invention concerne le domaine de la gestion des eaux usées dans les réseaux d'assainissement. Ces derniers sont soumis à un important problème : celui de la présence d'eaux parasites en plus (ou remplacement) des eaux usées. En effet, les réseaux d'assainissement sont normalement prévus pour la gestion des eaux usées et permettre leur traitement spécifique. En général, les eaux parasites sont quant à elles des eaux non chargées en pollution, ne nécessitant donc pas de traitement, et on les dénommes aussi eaux claires parasites. Ces eaux parasites peuvent être d'origine naturelle (captage de sources, drainage de nappes, fossés, inondations de réseaux ou de postes de refoulement, etc. ) ou artificielle (fontaines, drainage de bâtiments, eaux de refroidissement, rejet de pompe à chaleur, de climatisation, etc. ). Elles présentent l'inconvénient de diluer les effluents d'eaux usées et de réduire la capacité de transport disponible dans les réseaux d'assainissement et de saturer les stations d'épuration.

Ces eaux parasites, le plus souvent d'origine pluviale, peuvent aussi s'infiltrer et entrer dans les réseaux d'assainissement par le biais de fuites ou de défauts d'infrastructures. Ces infiltrations d'eaux parasites viennent gonfler les volumes d'eaux à traiter dans les stations d'épuration, parfois de manière conséquente. Or, les réseaux d'assainissement sont dimensionnés pour un certain nombre d'habitants dans une zone géographique définie et les eaux parasites peuvent conduire à un dépassement des capacités de traitement et les capacités de collecte des réseaux d'assainissement. En outre les eaux circulant dans les réseaux peuvent aussi transporter des matières solides pouvant former des dépôts qui vont réduire la capacité des réseaux.

Une solution rationnelle, écologique et économique consisterait à limiter fortement voir éliminer complètement les eaux parasites des réseaux d'assainissement. De même, il serait préférable d'empêcher que les matières solides entrent dans les réseaux, par exemple par mise en œuvre de bacs de décantation en amont.

Or, les études de terrain permettant de détecter ces eaux parasites restent à ce jour longues, couteuses et difficiles à mettre en place. Les moyens empêchant l'entrée des matières solides dans les réseaux représentent un coût supplémentaire et nécessitent une surveillance.

Il est donc particulièrement intéressant de développer des outils d'utilisation simple permettant de détecter et de quantifier simplement et efficacement les eaux parasites dans les différentes parties du réseau d'assainissement afin d'en identifier les sources et de pouvoir ensuite efficacement les éliminer.

Il en est de même pour les dépôts de matières solides ou sédiments qui viennent se déposer sur le fond nu du réseau d'assainissement. Le fond nu correspond au sol ou fond du réseau d'assainissement qui est sans dépôt ou sédiment.

Dans le cadre de la présente invention, il est proposé des moyens permettant de détecter, identifier/catégoriser et quantifier les différentes eaux pouvant circuler dans un réseau d'assainissement et en particulier les eaux parasites et les sédiments par des mesures de la conductivité des eaux.

En effet, les eaux parasites ayant une origine souvent pluviale, leur teneur en ions et donc leur conductivité est très faible, typiquement inférieure à 30 $\mu$S/cm. Les eaux usées ont quant à elles des conductivités élevées voir très élevées, 1 mS/cm en général et jusqu'à 10 mS/cm pour certaines eaux usées industrielles.

Le suivi de l'évolution de la conductivité de l'eau peut donc permettre de détecter la présence et de déterminer la proportion d'eaux parasites à un instant donné. Toutefois, cela ne permet pas de connaitre la quantité d'eaux parasites en valeur absolue. Il est donc aussi utile de pouvoir mesurer le débit global des eaux au point d'analyse.

La solution proposée pour cela, mesure de la hauteur/niveau des eaux, s'éloigne des moyens connus à capteur radar à ultrasons ou des sondes à effet doppler ou des capteurs de pression pour connaitre le niveau d'eau et le débit. En outre, ces moyens connus ne permettent pas de faire la distinction entre les divers types d'eau, dont les eaux parasites.

Dans la suite, le terme « eau » ou « eaux » seul, sans précision sur le type d'eau, correspond à tout type d'eau pouvant être rencontré au sein d'un réseau d'assainissement. L'eau d'un réseau d'assainissement peut par exemple comporter des eaux usées et/ou des eaux parasites.

On connait par le document FR2 701 566 un procédé de mesure d'au moins une caractéristique d'une solution liquide. On connait également les documents DE 26 43 964 A1, US 2010/295565 A1, US 2007/164751 A1, US 2011/048126 A1, US 2013/221986 A1 et US 2018/252569 A1.

**Résumé de l'invention**

La solution proposée par l'invention concerne un procédé mesure d'au moins un paramètre d'une eau d'un réseau d'assainissement, dans lequel on met en œuvre un capteur destiné à être disposé au contact de ladite eau et comportant en surface des électrodes, dont une électrode de référence et un nombre déterminé N supérieur à un d'électrodes d'excitation, l'électrode de référence étant allongée verticalement et les électrodes d'excitation étant étagées d'une manière équidistante entre elles et alignées verticalement parallèlement à l'électrode de référence, chaque électrode d'excitation définissant avec l'électrode de référence une paire d'électrodes, et, dans une phase de mesures, on mesure pour chaque paire d'électrodes du capteur un courant électrique local $I_i$ circulant et un voltage local $V_i$ appliqué, et on corrige les courants électriques locaux en ôtant une valeur de décalage Z de mesure de courant pour produire des courants électriques locaux corrigés, et on calcule des conductances locales $Co_i$ corrigées en utilisant les courants électriques locaux corrigés, et on calcule des conductivités locales $EC_{25i}$ en utilisant les produits des conductances locales $Co_i$ par des constantes de cellule $K_i$ et en utilisant une mesure de température T de ladite eau pour corriger un effet de la température sur les conductances locales, et dans lequel on détermine une hauteur relative $Hr_{(mesure)}$ d'eau en utilisant les conductivités locales $EC_{25i}$, et dans lequel on calcule une conductivité globale $EC_{25}$ de ladite eau par une fonction ayant au moins un paramètre qui est la hauteur relative $Hr_{(mesure)}$ déterminée de ladite eau.

Dans le cadre de l'invention, le terme « courant » est compris comme étant un courant électrique correspondant à une intensité électrique et dont l'unité est l'Ampère. De même, le terme « tension » est compris comme étant une tension électrique correspondant à un voltage et dont l'unité est le Volt.

Toujours dans le cadre de l'invention, le qualificatif de « local » se rapporte à un élément concernant une électrode d'excitation donnée ou une paire d'électrodes donnée parmi les paires d'électrodes du capteur.

D'autres caractéristiques avantageuses du procédé conforme à l'invention, prises individuellement ou selon toutes les combinaisons techniquement possibles, sont les suivantes :

- ledit au moins un paramètre mesuré est la conductivité globale ;
- chaque paire d'électrodes (et l'électrode d'excitation correspondante) est référencée par un indice déterminé $i$, l'indice $i$ étant un entier positif compris entre 0 et $N$-1, la paire d'électrodes d'indice $i$ =0 étant la paire d'électrodes la plus basse du capteur et la paire d'électrodes d'indice $N$-1 étant la paire d'électrode la plus haute du capteur et l'indice s'incrémentant de 1 d'une paire d'électrodes à la suivante en remontant le long de la hauteur du capteur,

- lors de la phase de mesure, on calcule la conductivité locale $EC_{25i}$ d'une paire d'électrodes avant de passer à la paire d'électrode suivante en remontant le long du capteur ;
- on calcule la conductance locale corrigée $Co_i$ par correction du courant électrique local $I_i$ mesuré en retirant une valeur de décalage Z de mesure de courant, soit $Co_i = (I_i - Z) / V_i$;

- on calcule les conductivités locales par $EC_{25i} = \dfrac{Co_i * K_i}{1 + ((T° - 25) * v)}$ avec $Co_i = (I_i - Z) / V_i$ où v est un coefficient de variation de la conductance électrique de l'eau en fonction de la température, de préférence $v = 0{,}02$ ;

- on calcule les conductivités locales par $EC_{25i} = \dfrac{Co_i * K_i}{1 + ((T° - 25) * 0{,}02)}$ avec $Co_i = (I_i - Z)/ V_i$ ;

- on calcule la conductivité locale $EC_{25i}$ directement à partir du courant électrique local $I_i$ et du voltage local $V_i$ mesurés sans passer par un calcul intermédiaire d'une conductance,
- une conductivité locale brute $EC_i$ est calculée par le produit d'une conductance locale corrigée $Co_i$ par une constante de cellule $K_i$ de la paire d'électrodes $i$ correspondante, soit $EC_i = Co_i * K_i$ où la conductance locale corrigée $Co_i$ est calculée en corrigeant le courant électrique local $I_i$ par une valeur de décalage Z de mesure de courant, soit $Co_i = (I_i - Z) / V_i$ ;
- on calcule la conductivité locale $EC_{25i}$ en corrigeant la conductivité locale brute $EC_i$ des effets de la température T de l'eau par un calcul ramenant la conductivité locale à une température de référence déterminée, la température de référence déterminée étant de préférence 25°C ;
- dans le cas d'une température de référence déterminée de 25°C on calcule la conductivité locale $EC_{25i}$ par

$EC_{25i} = \dfrac{CO_i * K_i}{1 + ((T° - 25) * v)}$ ou par $EC_{25i} = \dfrac{((I_i - Z) / V_i) * K_i}{1 + ((T° - 25) * v)}$ ou par $EC_{25i} = \dfrac{EC_i}{1 + ((T° - 25) * v)}$ où $v$ est un coefficient de variation de la conductance électrique de l'eau en fonction de la température, de préférence $v = 0{,}02$ ;

- l'eau du réseau d'assainissement comporte une eau parasite ;
- on met en œuvre un capteur qui comporte des moyens de mesure du courant électrique circulant dans les paires d'électrodes ;
- on met en œuvre un capteur qui comporte des moyens de mesure de la tension appliquée entre les électrodes des paires d'électrodes ;
- la mesure de la conductance locale corrigée est effectuée à courant constant ou, de préférence, à tension constante ;

- la valeur de décalage *Z* de mesure de courant est déterminée au cours d'une phase d'étalonnage ;
- la valeur de décalage *Z* de mesure de courant est une valeur *Z* commune à toutes les paires d'électrodes ;
- la valeur de décalage *Z* de mesure de courant est le courant électrique mesuré alors qu'aucune électrode d'excitation n'est reliée aux moyens de mesure de courant mesurant le courant électrique ;
- la valeur de décalage *Z* de mesure de courant est le courant électrique mesuré alors que le capteur est hors d'eau et sec ;
- la valeur de décalage *Z* de mesure de courant est la moyenne des courants électriques locaux $I_i$ mesuré sur l'ensemble des paires d'électrodes alors que le capteur est hors d'eau et sec ;
- on mesure la température T° de l'eau et le calcul de la conductivité locale est corrigé des effets de la température de l'eau en ramenant la conductivité locale calculée à une température de référence déterminée, la température de référence déterminée étant de 25°C, la formule de calcul corrigeant la température étant :

$$EC_{25i} = \frac{CO_i * K_i}{1 + ((T° - 25) * 0{,}02)}$$

avec : $EC_{25i}$ la conductivité locale à 25°C en S/cm pour la paire d'électrodes d'indice *i* , $Co_i$ la conductance locale corrigée pour la paire d'électrodes d'indice *i* , $K_i$ la constante de cellule de la paire d'électrodes d'indice *i*, T° la température mesurée en °C de l'eau ;
- lors de la mesure du courant électrique local $I_i$ et du voltage local $V_i$ on applique à la paire d'électrodes un voltage alternatif périodique de fréquence d'excitation déterminée, ledit voltage alternatif étant en forme de créneaux comportant des plateaux de voltages successifs de signes opposés ;
- on génère le voltage alternatif périodique par un pont-en-H ;
- le voltage alternatif périodique comporte des plateaux de voltages successifs de signes opposés et de même valeur absolue ;
- on mesure le courant électrique local $I_i$ et le voltage local $V_i$ pendant le plateau ;
- le voltage alternatif périodique comporte des plateaux de voltages successifs de signes opposés, de même valeur absolue et de même durée de chaque plateau afin que le voltage alternatif périodique ait un facteur de crête égal à 1 ;
- lors de la phase de mesure, la fréquence d'excitation déterminée est choisie dans un ensemble de fréquences de mesures en fonction d'une conductivité (locale ou globale selon le cas) de l'eau attendue ou supposée ou préalablement calculée ;
- l'ensemble de fréquences de mesures comporte trois fréquences qui sont 500 Hz, 1000 Hz et 5000 Hz ;
- on met en œuvre une fréquence d'excitation déterminée de 500 Hz pour une conductivité locale à mesurer inférieure ou égale à 1999 μS/cm, de 1.000 Hz pour une conductivité locale à mesurer comprise entre 2000 μS/cm et 9999 μS/cm, de 5.000 Hz pour une conductivité locale à mesurer supérieure ou égale à 10.000 μS/cm ;
- on met en œuvre une fréquence d'excitation déterminée de 500 Hz pour une conductivité globale à mesurer inférieure ou égale à 1999 μS/cm, de 1.000 Hz pour une conductivité globale à mesurer comprise entre 2000 μS/cm et 9999 μS/cm, de 5.000 Hz pour une conductivité globale à mesurer supérieure ou égale à 10.000 μS/cm ;
- si suite à une première mesure, un premier calcul de conductivité locale $EC_{25i}$ donne une valeur qui ne correspond pas aux conductivités prévues pour la fréquence d'excitation utilisée à ladite première mesure, alors on recommence la mesure avec une fréquence d'excitation adaptée à la conductivité obtenue à ladite première mesure ;
- si suite à une première mesure, un premier calcul de conductivité globale $EC_{25}$ donne une valeur qui ne correspond pas aux conductivités prévues pour la fréquence d'excitation utilisée à ladite première mesure, alors on recommence la mesure avec une fréquence d'excitation adaptée à la conductivité obtenue à ladite première mesure ;
- dans une phase d'étalonnage, on détermine, pour une fréquence déterminée, les constantes de cellule $K_i$ ;
- dans une phase d'étalonnage, on détermine, pour chaque fréquence de l'ensemble de fréquences de mesure, les constantes de cellule $K_i$ ;
- dans la phase de mesure on utilise les constantes de cellule $K_i$ correspondant à la fréquence d'excitation déterminée utilisée ;
- dans une phase d'étalonnage, on détermine une valeur de décalage *Z* de mesure de courant pour chaque fréquence de l'ensemble de fréquences de mesures ;
- on met en œuvre une détection de paires d'électrodes émergées, i.e. hors d'eau, une paire d'électrodes étant émergée si la conductivité locale $EC_{25i}$ pour ladite paire d'électrodes est nulle, c-à-d 0 μS/cm et que la ou les paires d'électrodes situées au-dessus de ladite paire d'électrodes a ou ont des conductivités locales nulles ;
- dans la phase de mesures, on effectue les mesures et les calculs des conductivités locales paire d'électrodes après paire d'électrodes en commençant par la paire d'électrodes la plus basse du capteur et en remontant d'une paire d'électrodes à la suivante le long de la hauteur du capteur ;
- lors de la phase de mesures on arrête les mesures et les calculs des conductivités locales lorsqu'un nombre déterminé de paires d'électrodes successives ont des conductivités locales nulles, ledit nombre déterminé étant deux ou plus de deux ;

- on met en œuvre une détection de paires d'électrodes défaillantes, une paire d'électrodes étant défaillante si la conductivité locale $EC_{25i}$ pour ladite paire d'électrodes est nulle alors qu'elle est surmontée par au moins une paire d'électrodes à conductivité locale non nulle, et on détermine le nombre m de paires d'électrodes défaillantes ;

- on met en œuvre une détection de paires d'électrodes défaillantes et on corrige le calcul de la hauteur relative $Hr_{(mesure)}$ pour tenir compte de la présence d'une ou plusieurs paires d'électrodes défaillantes ;

- lors de la phase de mesures on arrête les mesures lorsqu'un nombre déterminé de paires d'électrodes défaillantes a été détecté, ledit nombre déterminé étant de préférence un multiple entier de deux ou plus préférentiellement deux ;

- on calcule la hauteur relative $Hr_{(mesure)} = H_1$ d'eau par :

$$H_1 = (EC_{mean} * H_{max} / EC_{max})$$

avec :

$EC_{max}$ la conductivité locale $EC_{25i}$ la plus grande parmi les conductivités locales $EC_{25i}$ de l'ensemble des paires d'électrodes du capteur et

$EC_{mean}$ la conductivité moyenne mesurée sur toutes les paires d'électrodes du capteur, c-à-d la somme des conductivités locales $EC_{25i}$ divisée par le nombre total de paires d'électrodes du capteur, et

$H_{max}$ la hauteur relative maximale d'eau mesurable par le capteur ;

- la hauteur relative maximale d'eau mesurable par le capteur, $H_{max}$, est le produit du nombre $N$ d'électrodes d'excitation par e qui est le pas d'espacement entre deux électrodes d'excitation successives le long de la hauteur du capteur, soit $H_{max} = N * e$ ;

- pour le calcul de la hauteur relative $Hr_{(mesure)} = H_1$ on utilise les conductivités locales $EC_{25i}$ ;

- on calcule la hauteur relative $Hr_{(mesure)} = Ho_1$ d'eau par :

$$Ho_1 = (EC_{mean} * H_{max} / EC_{max}) + H_{corr}$$

avec :

$EC_{max}$ la conductivité locale $EC_{25i}$ la plus grande parmi les conductivités locales $EC_{25i}$ de l'ensemble des paires d'électrodes du capteur et

$EC_{mean}$ la conductivité moyenne mesurée sur toutes les paires d'électrodes du capteur dans la phase de mesures, c-à-d la somme des conductivités locales $EC_{25i}$ divisée par le nombre total de paires d'électrodes du capteur, et

$H_{max}$ la hauteur relative maximale d'eau mesurable par le capteur, et

$H_{corr}$ une valeur de correction de hauteur calculée par $H_{corr} = e * m$ où $m$ est le nombre de paires d'électrodes défaillantes et où e est le pas d'espacement entre deux électrodes d'excitation successives le long de la hauteur du capteur, c-à-d d'indices $i$ et $i+1$, la paire d'électrodes d'indice $i+1$ étant au-dessus de celle d'indice $i$ ;

- pour le calcul de la hauteur relative $Hr_{(mesure)} = Ho_1$ on utilise les conductivités locales $EC_{25i}$ ;

- on calcule la hauteur relative $Hr_{(mesure)} = H_2$ d'eau par:

$$H_2 = (I_{last} * e ) + ax^3 + bx^2 + cx + d$$

avec :

$$x = \frac{\frac{EC_{last}}{EC_{last-1}} + \frac{EC_{last-1}}{EC_{last-2}}}{2} ,$$

avec

$a, b, c, d$ des constantes prédéterminées,

e le pas d'espacement entre deux électrodes d'excitation successives le long de la hauteur du capteur, c-à-d d'indices $i$ et $i+1$, la paire d'électrodes d'indice $i+1$ étant au-dessus de celle d'indice $i$,

$EC_{last}$ la conductivité locale $EC_{25i}$ de la dernière paire d'électrodes vers le haut encore en contact avec l'eau, c-à-d la paire d'électrodes de conductivité locale non nulle la plus haute,

$I_{last}$ est l'indice de la dernière paire d'électrodes vers le haut encore en contact avec l'eau, la paire d'électrodes la plus basse du capteur ayant un indice égal à zéro ;

- dans le cas du calcul de la hauteur relative $Hr_{(mesure)} = H_2$, les constantes sont avantageusement les suivantes : 114 < a < 115, -217 < b < -216, 151 < c < 152 et - 31 < d < -30 ;
- pour le calcul de la hauteur relative $Hr_{(mesure)} = H_2$ on utilise les conductivités locales $EC_{25i}$ ;
- le pas d'espacement e entre deux électrodes d'excitation successives le long de la hauteur du capteur est mesuré entre les centres de deux électrodes d'excitation successives, i.e. d'indices i et i+1 ;
- le pas d'espacement e entre deux électrodes d'excitation successives le long de la hauteur du capteur est constant sur toute la hauteur du capteur ;
- on calcule la hauteur relative d'eau par $Hr_{(mesure)} = H_1$ ou par $Hr_{(mesure)} = Ho_1$ ou par $Hr_{(mesure)} = H_2$ en fonction d'un résultat de tests logiques où

si ( ($I_{last}$ < 2) ou ( ($I_{last}$ <$SEUIL_{capteur}$) et ( (si la paire d'électrodes d'indice $I_{last}$ est non défaillante et si la paire d'électrodes d'indice $I_{last}$ -1 est défaillante) ou (si la paire d'électrodes d'indice $I_{last}$ - 1 est non défaillante et si la paire d'électrodes d'indice $I_{last}$ - 2 est défaillante) ) ) ) alors la hauteur relative d'eau est calculée avec $Hr_{(mesure)} = H_1$ ou, de préférence, avec $Hr_{(mesure)} = Ho_1$ ,
sinon la hauteur relative d'eau est calculée avec $Hr_{(mesure)} = H_2$ ,
$I_{last}$ étant l'indice de la dernière paire d'électrodes vers le haut encore en contact avec le l'eau, la paire d'électrodes la plus basse du capteur ayant un indice égal à zéro, et
$SEUIL_{capteur}$ étant un paramètre fonction du nombre N d'électrodes d'excitation du capteur ;

- le paramètre $SEUIL_{capteur}$ est égal à 3/4 * N ;
- l'eau est présente sur un fond nu du réseau d'assainissement et on calcule une hauteur absolue $Ha_{(mesure)}$ d'eau dans le réseau d'assainissement par $Ha_{(mesure)} = Hr_{(mesure)} + H_{offset}$ avec :
$H_{offset}$ la hauteur entre le fond nu du réseau d'assainissement et le bas de l'électrode d'excitation de la paire d'électrode la plus basse du capteur, i.e. la paire d'électrodes d'indice i = 0 ;
- on calcule la conductivité globale $EC_{25}$ par $EC_{25} = ECmoyen_{(Hmax)}$ avec

$$ECmoyen_{(Hmax)} = EC_{mean} * H_{max} / Hr_{(mesure)}$$

où :

$EC_{mean}$ est la conductivité moyenne mesurée sur toutes les paires d'électrodes du capteur dans la phase de mesures, c-à-d la somme des conductivités locales $EC_{25i}$ divisée par le nombre total de paires d'électrodes du capteur, et
$Hr_{(mesure)}$ est la hauteur relative d'eau , et
$H_{max}$ est la hauteur relative maximale d'eau mesurable par le capteur ;

- dans une phase d'étalonnage, on détermine avec le capteur une courbe d'évolution de la conductivité moyenne mesurée sur toutes les paires d'électrodes en fonction d'une hauteur réelle relative d'eau mesurée par un dispositif de mesure, ledit dispositif étant distinct du capteur et étant notamment un radar, et on détermine une droite ajustée à ladite courbe et à partir de ladite droite on détermine une valeur Dk de décalage de conductivité moyenne, ladite valeur Dk étant la conductivité moyenne à hauteur réelle relative nulle d'eau sur la droite ajustée à la courbe, et dans lequel, dans la phase de mesure, on corrige le calcul de la conductivité globale $EC_{25}$ avec la valeur Dk de décalage de conductivité moyenne par :

$$EC_{25} = ECmoyen_{(Hmax)} = Dk + (EC_{mean} - Dk) * H_{max} / Hr_{(mesure)} ;$$

- le radar est un dispositif indépendant du système et est utilisé pour un étalonnage ;
- le capteur ne comporte pas de radar ;
- le système ne comporte pas de radar ;
- la hauteur réelle relative d'eau est mesurée par un dispositif de mesure de hauteur qui est un radar ;
- le radar est un radar à ultrasons ;
- pour le calcul de la conductivité globale $EC_{25} = ECmoyen_{(Hmax)}$ on utilise les conductivités locales $EC_{25i}$ ;
- on calcule la conductivité globale $EC_{25}$ par $EC_{25} = EC_{eau}$ avec $EC_{eau} = EC_{max} / \propto$ où $EC_{max}$ est la conductivité locale la plus grande parmi les conductivités locales de l'ensemble des paires d'électrodes du capteur et

$\propto$ qui est un coefficient de corrélation fonction de la hauteur relative $Hr_{(mesure)}$ d'eau et ayant une valeur comprise entre 0 et 1, $\propto$ étant calculé par :

$\propto = a_1 * \ln(b_1 * Hr_{(mesure)}) + c_1 * Hr_{(mesure)}^3 + d_1 * Hr_{(mesure)}^2 + e_1 * Hr_{(mesure)} + f_1$ avec $a_1, b_1, c_1, d_1, e_1$ et $f_1$ qui sont des constantes prédéterminées ;

- dans le cas du calcul de la conductivité globale $EC_{25} = EC_{eau}$ , les constantes sont avantageusement les suivantes : 5,4E-01 < $a1$ < 5,5E-01, 1,8E+06 < $b1$ < 1,9E+06, - 3,9E-08 < $c1$ < -3,8E-08, 2,9E-05 < $d1$ < 3,0E-05, 9,5E-03 < $e1$ < 9,6E-03 et 8,7E+00 < $f1$ < 8,8E+00;
- pour le calcul de la conductivité globale $EC_{25} = EC_{eau}$ on utilise les conductivités locales $EC_{25i}$ ;
- pour le calcul de la conductivité globale $EC_{25}$ en fonction de la hauteur relative $Hr_{(mesure)}$ on utilise soit $Hr_{(mesure)} = H_1$ , soit $Hr_{(mesure)} = Ho_1$ , soit $Hr_{(mesure)} = H_2$ ;
- on calcule une salinité S de l'eau en fonction de la température T de l'eau et de la conductivité globale $EC_{25}$ ;
- on calcule la salinité S de l'eau par :

$S = a0 + (a1 * Rt^{0,5}) + (a2 * Rt) + (a3 * Rt^{1,5}) + (a4 * Rt^2) + (a5 * Rt^{2,5}) + ((T - 15)/(1 + k(T - 15))) * (b0 + (b1 * Rt^{0,5}) + (b2 * Rt) + (b3 * Rt^{1,5}) + (b4 * Rt^2) + (b5 * Rt^{2,5}))$

avec :

$$Rt = (EC_{25}/42{,}914) / (c0 + (c1 * T) + (c2 + T^2) + (c3 * T^3) + (c4 * T^4))$$

et

| | | |
|---|---|---|
| a0 = 0,0080 | b0 = 0,0005 | c0 = 0,6766097 |
| a1 = -0,1692 | b1 = -0,0056 | c1 = 0,0200564 |
| a2 = 25,3851 | b2 = -0,0066 | c2 = 0,000110426 |
| a3 = 14,0941 | b3 = -0,0375 | c3 = -6,9698E-07 |
| a4 = -7,0261 | b4 = 0,0636 | c4 = 1,0031E-09 |
| a5 = 2,7081 | b5 = -0,0144 | k = 0,0162 |

où la conductivité globale $EC_{25}$ de l'eau est en mS/cm et $T$ est la température en °C ;
- on met en œuvre une détection de sédiments, une paire d'électrodes d'indice $i$ étant soumise à des sédiments si sa valeur de conductivité locale $EC_{25i}$ est non nulle et est soit inférieure à $EC_{25i+1} * (1 - Pk)$ , soit supérieure à $EC_{i+1} * (1 + Pk)$, où $Pk$ est un facteur de détection choisi entre 0,1 et 0,9, et dans lequel on calcule une hauteur relative de sédiments $Hr_{sediments}$ par le produit de la valeur d'indice $i$ plus 1 par $e$ qui est le pas d'espacement entre deux électrodes d'excitation successives le long de la hauteur du capteur, soit $Hr_{sediments} = e * (i + 1)$ ;
- on met en œuvre une détection de paires d'électrodes défectueuses ;
- on met en œuvre une détection de paires d'électrodes défectueuses, une paire d'électrodes d'indice $i$ étant défectueuse si sa valeur de conductivité locale $EC_{25i}$ est non nulle et en dehors d'une gamme de valeurs définie par les deux bornes $EC_{25i+1} * (1 - Ps)$ et $EC_{i+1} * (1 + Ps)$ et si $EC_{25i+1}$ est non nulle où $Ps$ est un facteur de sensibilité choisi entre 0,1 et 0,9 ;
- on met en œuvre une détection de type d'encrassement d'électrode ;
- on met en œuvre une détection de type d'encrassement d'électrode parmi la/les paires d'électrodes défectueuses ;
- la détection de paires d'électrodes encrassées à type isolant met en œuvre des calculs et comparaisons semblables à celles mises en œuvre pour la détection de sédiments mais avec un facteur de détection $Pk$ différent ;
- lors de la mesure du courant électrique local $I_i$ et du voltage local $V_i$ on applique à la paire d'électrodes un voltage alternatif périodique de fréquence d'excitation déterminée, ledit voltage alternatif étant en forme de créneaux comportant des plateaux de voltages successifs de signes opposés ;
- lors de la phase de mesure, la fréquence d'excitation déterminée est choisie dans un ensemble de fréquences de mesures en fonction d'une conductivité présupposée ou préalablement calculée de l'eau ;
- on génère le voltage alternatif périodique par un pont-en-H ;
- le voltage alternatif périodique comporte des plateaux de voltages successifs de signes opposés et de même valeur absolue ;
- le voltage alternatif périodique comporte des plateaux de voltages successifs de signes opposés, de même valeur absolue et de même durée de chaque plateau afin que le voltage alternatif périodique ait un facteur de crête égal à 1 ;
- dans une phase d'étalonnage, on détermine, pour chaque fréquence de l'ensemble de fréquences de mesure, les constantes de cellule $K_i$ ;
- dans la phase de mesure on utilise les constantes de cellule $K_i$ correspondant à la fréquence d'excitation déterminée utilisée ;

- dans une phase d'étalonnage, on détermine une valeur de décalage $Z$ de mesure de courant.

La solution proposée par l'invention porte également sur un système de mesure spécialement configuré pour assurer la mise en œuvre du procédé de l'invention. Plus précisément, l'invention concerne un système de mesure d'au moins un paramètre d'une eau d'un réseau d'assainissement, le système comportant un pont-en-H et des moyens matériels dont de calculs, en particulier un microcontrôleur à programme de contrôle spécialement configuré pour assurer la mise en œuvre du procédé décrit et comportant un pont-en-H.

D'autres caractéristiques avantageuses du système conforme à l'invention, prises individuellement ou selon toutes les combinaisons techniquement possibles, sont les suivantes :

- le système comporte des moyens matériels dont de calculs permettant d'exécuter une ou plusieurs actions, e.g. déterminations et calculs, du procédé de l'invention ;
- le système destiné à mesure d'au moins un paramètre d'une eau d'un réseau d'assainissement comporte un capteur comportant en surface des électrodes, dont une électrode de référence et un nombre $N$ déterminé d'électrodes d'excitation, l'électrode de référence étant allongée verticalement et les électrodes d'excitation étant étagées d'une manière équidistante entre elles et alignées verticalement parallèlement à l'électrode de référence, chaque électrode d'excitation définissant avec l'électrode de référence une paire d'électrodes de mesure indicée $i$, l'indice $i$ étant un entier compris entre 0 et $N$-1, la paire d'électrodes indicée 0 étant la paire d'électrodes du bas du capteur, le système comportant en outre un ensemble électronique comportant un circuit générateur de tension constante, un pont-en-H, un circuit de mesure de courant d'alimentation de pont, un circuit de mesure de tension d'alimentation de pont, un démultiplexeur analogique, un microcontrôleur à programme de contrôle,

    le système comportant en outre un circuit de mesure de température de l'eau et le microcontrôleur à programme de contrôle est configuré pour en outre acquérir numériquement la mesure de température,

    le pont-en-H comportant une entrée d'alimentation ayant deux connexions d'alimentation recevant une tension d'alimentation de pont, une sortie de pont ayant deux connexions de sortie produisant entre elles une tension de sortie de pont et une entrée de commande de pont, le pont-en-H permettant, en fonction de la commande de pont, au moins d'obtenir entre les deux connexions de sortie une tension de sortie de pont positive ou négative et de valeur absolue sensiblement égale à ladite tension d'alimentation de pont,

    le démultiplexeur comportant un port de sortie analogique, un ensemble de ports d'entrées analogiques et une entrée de commande de sélection d'entrée permettant en fonction de la commande de sélection d'entrée de relier ou non, un seul à la fois, le port d'entrée sélectionné au port de sortie, chacune des électrodes d'excitation étant reliée à un port d'entrée correspondant du démultiplexeur,

    l'électrode de référence étant reliée à une première des deux connexions de sortie du pont-en-H et chacune des électrodes d'excitation pouvant être reliée par l'intermédiaire du port de sortie du démultiplexeur à la seconde des deux connexions de sortie du pont-en-H,

    le circuit générateur de tension constante fournissant la tension d'alimentation de pont par l'intermédiaire du circuit de mesure de courant d'alimentation de pont,

    le circuit de mesure de courant d'alimentation de pont mesurant le courant destiné à l'entrée d'alimentation du pont-en-H afin d'obtenir une mesure du courant électrique local $I_i$ circulant dans la paire d'électrodes sélectionnée $i$ par le démultiplexeur, le circuit de mesure de tension d'alimentation de pont mesurant la tension d'alimentation du pont-en-H afin d'obtenir une mesure du voltage $V_i$ appliqué entre les deux électrodes de la paire d'électrodes sélectionnée par le démultiplexeur ;

- le microcontrôleur à programme de contrôle est configuré pendant la mesure de ladite au moins une caractéristique de l'eau du réseau d'assainissement, pour :

    -- dans une première étape : commander l'entrée de commande de sélection d'entrée du démultiplexeur pour relier la sortie du pont-en-H à une électrode d'excitation sélectionnée pour mesure parmi les paires d'électrodes,

    -- dans une deuxième étape : commander régulièrement l'entrée de commande de pont par une commande de basculement afin que la tension de sortie de pont puisse basculer en positive et négative à une fréquence d'excitation définie, et une fois la tension de sortie de pont basculée, acquérir numériquement au moins une mesure de courant d'alimentation de pont provenant du circuit de mesure de courant d'alimentation de pont, et acquérir numériquement au moins une mesure de tension d'alimentation de pont provenant du circuit de mesure de tension d'alimentation de pont, puis

    -- dans une troisième étape : calculer, en fonction de ladite au moins une mesure de courant d'alimentation de pont acquise, de ladite au moins une mesure de tension d'alimentation de pont acquise et d'une mesure de température acquise, une conductance locale corrigée $Co_i$ et une conductivité locale $EC_{25i}$ ou directement une conductivité locale $EC_{25i}$ pour la paire d'électrodes d'indice $i$ sélectionnée par le démultiplexeur ;

- le microcontrôleur à programme de contrôle est configuré pour itérer les première, deuxième et troisième étape afin d'obtenir les une conductivité locale $EC_{25i}$ de chacune des paires d'électrodes ;
- la tension constante est une tension continue ;
- le microcontrôleur à programme de contrôle est configuré pour que la tension de sortie du circuit générateur de tension constante soit nulle en dehors de la mesure dudit au moins un paramètre de l'eau du réseau d'assainissement ;
- le microcontrôleur à programme de contrôle est configuré pour que la tension de sortie du circuit générateur de tension constante soit nulle tant que la commande régulière de l'entrée de commande de pont n'est pas commencée et dès que la/les acquisitions de la/des mesures de courant d'alimentation de pont et de la/des mesures de tension d'alimentation de pont sont terminées,
- le circuit générateur de tension constante est contrôlé par le microcontrôleur, la tension de sortie dudit circuit générateur pouvant être rendue nulle ou avoir une valeur constante déterminée selon une commande du programme de contrôle, et le programme de contrôle amène la tension de sortie dudit circuit générateur à la valeur constante déterminée juste après la commande de basculement et avant les acquisitions des mesures de courant et tension, puis amène la tension de sortie dudit circuit générateur à la valeur nulle juste après les acquisitions des mesures de courant et tension ;
- le microcontrôleur à programme de contrôle est configuré pour corriger la mesure de courant d'alimentation de pont acquise en ôtant à la mesure un courant de fuite $Z$ dû au pont-en-H, ledit courant de fuite étant une valeur de décalage $Z$ de mesure de courant obtenue au cours d'une phase d'étalonnage dans laquelle le démultiplexeur ne relie pas le pont-en-H à une des paires d'électrodes ;
- le microcontrôleur à programme de contrôle est configuré pour corriger la mesure de courant d'alimentation de pont acquise en ôtant à la mesure un courant de fuite $Z$ dû au pont-en-H, au démultiplexeur et éventuellement à une paire d'électrodes sélectionnée (i.e. reliée au pont-en-H), le courant de fuite étant une valeur de décalage $Z$ de mesure de courant obtenue au cours d'une phase d'étalonnage dans laquelle le capteur est à sec, les paires d'électrodes n'étant pas immergées au contact de l'eau ;
- dans la deuxième étape, les acquisitions sont synchronisées avec les commandes de basculement de la tension de sortie de pont et les acquisitions sont effectuées après un délai déterminé suivant une commande de basculement de la tension de sortie de pont afin que ladite tension de sortie de pont soit stabilisée pendant les acquisitions ;
- le démultiplexeur analogique est bidirectionnel ;
- le pont-en-H comporte deux circuits push-pull entre les deux connexions d'alimentation et les deux connexions de sortie sont aux deux points milieux des deux circuits push-pull ;
- la tension de sortie de pont qui bascule en positive et négative à une fréquence d'excitation définie est un signal carré de valeur moyenne nulle sur une période ;
- la tension de sortie de pont qui bascule en positive et négative à une fréquence d'excitation définie est un signal carré dont la durée de tension positive est égale à la durée de tension négative sur une période ;
- le pont-en-H permet en outre, en fonction de la commande de pont, d'appliquer ou non la tension d'alimentation sur la sortie ;
- le pont-en-H permet en outre, en fonction de la commande de pont, de rendre la sortie flottante (i.e. haute impédance) ;
- les entrées du démultiplexeur peuvent être toutes rendues flottantes (i.e. haute impédance) ;
- une première des deux connexions d'alimentation du pont-en-H est reliée à un potentiel commun de masse de l'ensemble électronique, la seconde connexion d'alimentation du pont-en-H étant reliée au circuit générateur de tension constante par l'intermédiaire du circuit de mesure de courant, et le circuit de mesure de tension d'alimentation de pont mesure la tension entre la seconde connexion d'alimentation du pont-en-H et le potentiel commun de masse de l'ensemble électronique ;
- le microcontrôleur à programme de contrôle comporte au moins un convertisseur analogique numérique (i.e. analogique vers numérique) ;
- la mesure de courant d'alimentation de pont issue du circuit de mesure de courant d'alimentation de pont est acquise par un convertisseur analogique numérique du microcontrôleur ;
- la mesure de tension d'alimentation de pont issue du circuit de mesure de tension d'alimentation de pont est acquise par un convertisseur analogique numérique du microcontrôleur ;
- le microcontrôleur à programme de contrôle comporte au moins un convertisseur numérique analogique (i.e. numérique vers analogique) ;
- la tension de sortie du circuit générateur de tension constante est sous le contrôle du microcontrôleur, ladite tension de sortie pouvant être rendue nulle ou avoir une valeur constante déterminée sélectionnable par le programme de contrôle ;
- les électrodes d'excitation et de référence sont agencées sur un matériau hydrophobe et non conducteur de l'électricité ;
- les électrodes d'excitation et de référence sont agencées sur un matériau rigide, les électrodes étant rigides et le capteur est rigide ;
- les électrodes d'excitation et de référence sont agencées sur un matériau souple, les électrodes étant souples ;
- les électrodes sont électriquement isolées entre elles sur le capteur ;
- chaque électrode d'excitation est une électrode ponctuelle, localisée, de surface réduite par rapport à la surface de l'électrode de référence ;
- l'ensemble de ports d'entrées analogiques du démultiplexeur comporte un nombre de ports égal ou supérieur au nombre déterminé d'électrodes d'excitation ;
- l'ensemble de ports d'entrées analogiques du démultiplexeur comporte un nombre de ports égal au nombre déterminé d'électrodes d'excitation ;
- les électrodes d'excitation sont alignées et étagées en hauteur selon un pas constant, les électrodes d'excitation adjacentes étant séparées d'une distance identique ;
- les électrodes d'excitation sont alignées et étagées en hauteur selon un pas variable, les électrodes d'excitation adjacentes étant séparées d'une distance variant en fonction de la hauteur le long de la hauteur du capteur ;
- l'ensemble électronique comporte en outre au moins un circuit de mémoire numérique relié au microcontrôleur ;
- la mémoire du circuit de mémoire est une mémoire persistante ;
- le circuit de mémoire comporte des données numériques dont au moins les constantes de cellules $K_i$ et la valeur de décalage $Z$ de mesure de courant ;
- le microcontrôleur à programme de contrôle est en outre configuré pour calculer une hauteur relative $Hr_{(mesure)}$ de l'eau et, éventuellement, une hauteur absolue $Ha_{(mesure)}$ de l'eau, en fonction des conductivités locales $EC_{25i}$ calculées pour chacune des paires d'électrodes du capteur ;
- le microcontrôleur à programme de contrôle est en outre configuré pour calculer une conductivité globale $EC_{25}$ par une fonction utilisant la hauteur relative $Hr_{(mesure)}$ de l'eau ;
- le microcontrôleur à programme de contrôle est configuré pour utiliser une fréquence d'excitation définie de basculement de la tension de sortie de pont qui est de 500 Hz lorsque la plage de conductivité à mesurer est inférieure ou égale à 1999 µS/cm, de 1.000 Hz lorsque la plage de conductivité à mesurer est comprise entre 2000 µS/cm et 9999 µS/cm, de 5.000 Hz lorsque la plage de conductivité à mesurer est supérieure ou égale à 10.000 µs/cm.

**Brève description des figures**

Sur les figures annexées la :

[Fig. 1] représente une face avant de capteur destinée à recevoir une électrode de référence et des électrodes d'excitation dans un exemple de capteur utilisable dans l'invention,

[Fig. 2] représente une électrode de référence pour la face avant de l'exemple de capteur de la figure 1,

[Fig. 3] représente une vue en coupe axiale d'une électrode d'excitation pour la face avant de l'exemple de capteur de la figure 1,

[Fig. 4] représente une vue en perspective de l'arrière d'une électrode d'excitation pour la face avant de l'exemple de capteur de la figure 1,

[Fig. 5] représente en vue latérale, un dispositif à capteur comportant l'exemple de capteur utilisable dans l'invention et un support d'adaptation permettant une fixation et un réglage du dispositif sur une bordure de cunette,

[Fig. 6] représente en vue latérale, le dispositif à capteur de la figure 5 installé sur une bordure de cunette,

[Fig. 7] représente schématiquement le système de l'invention dans un exemple de réalisation où l'ensemble électronique est séparé en deux parties reliées entre elles et où le capteur comporte de 16 électrodes d'excitation,

[Fig. 8] représente l'évolution des conductivités locales calculées pour un ensemble de 16 électrodes d'excitation en fonction de l'évolution d'un niveau d'eau auquel le système est soumis, la hauteur absolue calculée du niveau d'eau étant aussi représentée,

[Fig. 9] représente l'évolution des conductivités locales calculées pour une partie des électrodes d'excitation en fonction de l'évolution d'un niveau d'eau auquel le système est soumis, certaines des électrodes d'excitation étant encrassées,

[Fig. 10] représente schématiquement l'ensemble électronique du système de l'invention,

[Fig. 11] représente schématiquement un pont-en-H tel que mis en œuvre dans l'ensemble électronique du système de l'invention et comportant deux connexions de sortie A et B et deux connexions d'alimentation 22a et 22b,

[Fig. 12] représente schématiquement le pont-en-H tel que mis en œuvre dans l'ensemble électronique du système de l'invention, pour les deux polarités opposées, positive et négative, produites entre les deux connexions de sortie A et B, et

[Fig. 13] représente sous forme d'un diagramme de flux les étapes permettant une mesure au sein du système de l'invention.

**Description détaillée**

La description qui va suivre en regard des dessins annexés, donnés à titre d'exemples non limitatifs, fera bien comprendre en quoi consiste l'invention et comment elle peut être réalisée.

Tout d'abord, il convient de noter que lorsqu'on mentionne (pour des raisons de simplification) une mesure sur ou pour ou d'une électrode d'excitation cela correspond à une mesure basée sur la mesure du courant circulant entre l'électrode d'excitation en question et l'électrode de référence, les deux électrodes définissant une paire d'électrodes de mesure.

En outre, du fait de l'utilisation d'un capteur comportant de multiples électrodes d'excitation formant chacune, avec l'électrode de référence, une paire d'électrodes, chaque paire d'électrodes et électrode d'excitation correspondante est identifiée par un numéro ou valeur d'indice $i$ pour faciliter la compréhension, l'indice i prenant une valeur comprise entre 0 et $N$-1 ($i \in [0, ..., N$-1] où N est le nombre d'électrodes d'excitation) selon l'électrode d'excitation considérée. Par convention pour les explications du fonctionnement de l'invention, l'indice $i = 0$ correspond à la paire d'électrodes la plus basse du capteur, i.e. électrode d'excitation ou paire d'électrodes numéro 0.

A chaque paire d'électrodes sont associés des mesures d'un courant électrique local et d'une tension locale ainsi qu'à des résultats de calculs (e.g. conductance locale et conductivité locale) sont aussi identifiés par un indice $i$ qui est le même que celui de la paire d'électrodes à l'origine des mesures et calculs. Cela permet aussi d'attribuer à une paire d'électrodes spécifique un résultat d'une détermination (e.g. est- ce une paire d'électrode défaillante ou défectueuse ? est-ce la paire d'électrode immergée la plus haute ? ...)

D'une façon générale, on qualifie de « local » ce qui concerne individuellement chaque paire d'électrodes et de « global » ce qui concerne le capteur globalement, c-à-d ce qui résulte de la mise en œuvre globale des paires d'électrodes, notamment résultant de calculs avec toutes ou un certain nombre supérieur à un de paires d'électrodes. Ainsi, un paramètre (e.g. courant, voltage, conductance, conductivité, valeur correctrice) indicé $i$ correspond à un paramètre local associé à une paire d'électrodes déterminée indicée $i$ (ou, encore, de numéro $i$).

En outre, le qualificatif « relatif » est utilisé pour indiquer que le capteur sert de référence. Ainsi, dans le cas de la hauteur relative $Hr_{(mesure)}$, le zéro de la mesure correspond au bord inférieur de l'électrode d'excitation la plus basse dudit capteur, i.e. d'indice égal à 0, sachant que le bord inférieur de l'électrode de référence est de niveau avec le bord inférieur de l'électrode d'excitation la plus basse.

A noter aussi que le bord supérieur de l'électrode de référence est de niveau avec le bord supérieur de l'électrode d'excitation la plus basse.

Le système et procédé de l'invention mettent en œuvre des mesures et calculs de la conductance afin de pouvoir calculer la conductivité d'une eau, typiquement des eaux usées d'un réseau d'assainissement pouvant comporter ou être remplacées par des eaux parasites, c-à-d non prévues pour s'y trouver.

Pour la mesure de la conductance on peut utiliser un capteur à deux électrodes appelées anode et cathode et on peut alors déterminer la valeur de la résistivité d'une eau, Rsol, par deux méthodes, soit à courant constant, soit à tension constante.

Pour la méthode à courant constant on envoie un courant I constant et connu à travers l'anode et on mesurer la tension V entre l'anode et la cathode. Pour la méthode à tension constante on applique une tension V constante et connue entre l'anode et la cathode et on mesure le courant I obtenu.

C'est cette dernière méthode qui est mise en œuvre dans le cadre de l'invention. On peut alors calculer la résistivité d'une l'eau, Rsol, en utilisant la loi d'Ohm. La conductance étant l'inverse de la résistivité de l'eau, Rsol, on peut calculer la conductance en considérant l'inverse du calcul de la résistivité, soit conductance = I/V.

La conductivité d'une eau quant à elle peut être directement obtenue à partir d'une mesure de la conductance ou directement obtenue à partir du courant I et de la tension V (donc sans passer par un calcul intermédiaire de la conductance). Ces deux possibilités peuvent être mises en œuvre dans le cadre des calculs de l'invention.

Les valeurs de résistivité et conductance obtenues dépendent toutefois des conditions de mesure et de la qualité de la chaîne de mesure.

En particulier, les électrodes des différents capteurs possèdent des tailles, formes, écartements possiblement différents et peuvent être dans des matériaux différents, ce qui va conduire à des valeurs différentes de conductance et surtout de conductivité pour une même eau entre différents capteurs.

En outre, la conductance varie en fonction de la température de l'eau.

Il est donc avantageusement mis en œuvre des moyens de calcul pour corriger les mesures de conductance et pour standardiser et uniformiser les valeurs de conductivités obtenues.

A cette fin, on met en œuvre dans l'invention un paramètre de correction des variations des mesures dues aux différences entre les électrodes et capteurs, ce paramètre étant appelé constante de cellule est spécifique de chaque paire d'électrodes dans le cadre de la présente invention. Ainsi une constante de cellule de la paire d'électrodes indicée $i$ est notée $K_i$ dans la suite.

Les constante de cellules $K_i$ sont donc des paramètres de correction de l'hétérogénéité des paires d'électrodes des capteurs.

Les constante de cellules $K_i$ servent à corrigent les conductances locales mesurées par le capteur pour obtenir les conductivités locales.

On met donc en œuvre un paramètre de correction des variations des mesures dues aux différences entre les électrodes et capteurs, ce paramètre étant appelé constante de cellule et noté $K_i$ dans la suite, l'indice $i$ résultant que chaque paire d'électrodes (indicée $i$) possède une constante de cellule qui lui est spécifique. Ainsi, dans le cadre de l'invention mettant en œuvre un capteur avec de multiples électrodes d'excitation (soit autant de paires d'électrodes), on prévoit une opération d'étalonnage du capteur permettant d'obtenir les constantes de cellules $K_i$ pour chacune des électrodes d'excitation (i.e. chacune des paires d'électrodes), ces constantes pouvant être différentes d'une électrode d'excitation à une autre et d'un capteur à l'autre. Les constantes de cellules $K_i$ obtenues lors de l'opération d'étalonnage sont de préférence stockées dans une mémoire persistante, i.e. non volatile, du système afin de pouvoir être utilisé lors des mesures ultérieures sans avoir à refaire des opérations d'étalonnage et de préférence dans une mémoire persistante au sein du capteur lui-même puisque ces constantes de cellules sont spécifiques du capteur étalonné.

Les constantes de cellules $K_i$, sont donc déterminées pour chaque capteur et pour chaque paire d'électrodes (i.e. électrode d'excitation sélectionnée et électrode de référence), au cours d'une phase d'étalonnage dans laquelle on utilise une solution étalon de valeur de conductance connue et permettant d'obtenir $K_i = \dfrac{\text{ConductanceEtalon}}{\text{ConductanceMesurée}}$ pour la paire d'électrodes d'indice i sélectionnée.

On verra qu'il est avantageux d'obtenir ces constantes de cellules $K_i$, pour chacune des fréquences d'excitation pouvant être utilisée dans le système.

On peut mettre en outre en œuvre une correction des mesures de la tension locale $V_i$ et du courant électrique local $I_i$.

Dans la mise en œuvre décrite, on corrige uniquement le courant électrique local $I_i$. Concernant la tension locale $V_i$ qui n'est pas mesurée directement aux bornes de la paire d'électrodes sélectionnée, on considère qu'étant donné le faible courant circulant dans l'eau, entre les électrodes, les chutes de tension dans le circuit amenant le courant à la paire d'électrodes sont négligeables. Dans une autre modalité de mise en œuvre on prévoit en outre une correction de ces chutes de tension.

Concernant donc la correction des mesures du courant électrique local $I_i$, on effectue un étalonnage pour déterminer une, $Z$, valeur de décalage de mesure de courant permettant d'effectuer par calcul une mise à zéro des courants de fuite pouvant exister au sein de la chaîne de mesure du système. Ces courants de fuite sont, par exemple, les courants de fuite des semi-conducteurs et des capacités mis en œuvre au sein de la chaîne de mesure du système et/ou les courants de fuite entre des électrodes. On met en œuvre une seule valeur $Z$ de décalage de mesure de courant pour chaque fréquence de mesure.

Ainsi, dans la mise en œuvre décrite, on corrige chaque courant électrique local $I_i$ par une seule valeur $Z$ de décalage de mesure de courant pour la fréquence de mesure, ladite valeur $Z$ ayant été déterminée au cours d'une phase d'étalonnage. Comme précédemment, les valeurs $Z$ de décalage de mesure de courant sont de préférence stockées dans une mémoire persistante du système afin de pouvoir être utilisées lors des mesures ultérieures.

De plus, s'il est préférable d'effectuer les mesures à une température de l'eau qui correspond à celle utilisée pour l'étalonnage, en particulier l'étalonnage avec la solution étalon, typiquement 25°C, on peut plus avantageusement mettre en œuvre une correction des effets de la température par calcul.

Ainsi, pour le calcul des conductivités aussi bien locales que globale, on prend en compte la température lors des mesures à cause de la forte influence de la température sur la conductivité et la conductance.

En général, l'effet de la température est non linéaire. Toutefois, dans le cadre de la mise en œuvre de l'invention avec une eau d'un réseau d'assainissement, la plage de température rencontrée est relativement limitée et on admet généralement que l'effet de la température est linéaire dans ladite plage et que la variation de la conductivité est de 2% par degré Celsius.

Ainsi, afin de standardiser les résultats des mesures au regard des effets de la température, il a été choisi une température de référence pour fournir les résultats des mesures et calculs des conductivités locales et globale. La température de référence la plus souvent admise est 25°C et on parle alors de conductivité à 25°C (ici : conductivités locales $EC_{25i}$ et conductivité globale $EC_{25}$).

C'est cette température de référence à 25°C qui a été choisie dans le cadre de l'invention mais on comprend bien qu'on peut simplement appliquer les moyens de l'invention à d'autres valeurs de température de référence et/ou d'autres taux de variation de la conductivité.

Pour l'eau d'un réseau d'assainissement, on met en œuvre dans le cadre de l'invention un calcul de conductivité locale à la température de référence de 25°C et avec correction par constante de cellule $K_i$ selon la formule :

$$EC_{25i} = \frac{CO_i * K_i}{1 + ((T° - 25) * 0{,}02)}$$

avec $EC_{25i}$ la conductivité locale à 25°C en S/cm pour la paire d'électrodes d'indice *i* ; $Co_i$ la conductance locale corrigée pour la paire d'électrodes d'indice *i*, $K_i$ la constante de cellule de la paire d'électrodes d'indice *i*, T° la température mesurée en °C de ladite eau et $Co_i = (I_i - Z) / V_i$ où $I_i$ est le courant électrique local circulant dans la paire d'électrodes indicée *i* et $V_i$ le voltage appliqué entre les électrodes de ladite paire d'électrodes indicée *i*, le courant et la tension étant mesurés et acquis dans le système.

C'est ce calcul de la conductivité locale $EC_{25i}$ avec prise en compte de la température qui est mis en œuvre pour le calcul de la hauteur relative $Hr_{(mesure)}$ de l'eau et de la conductivité globale $EC_{25}$ de l'eau.

Il est aussi mis en œuvre dans le cadre de l'invention une excitation des électrodes par une tension alternative lors des mesures, ce qui provoque des inversions du courant circulant entre les deux électrodes de la paire d'électrodes.

En effet, lorsqu'on applique une tension continue entre une anode et une cathode dans un milieu liquide conducteur, le courant circulant produit une électrolyse qui va créer une polarisation qui va rendre rapidement inopérantes les électrodes.

Afin d'éviter cet effet de polarisation, un pont-en-H est utilisé dans l'ensemble électronique du système et il va envoyer par sa sortie une tension alternative sur les électrodes pendant les mesures. Cette tension est un signal carré, constitué de créneaux positifs et négatifs de même durées et de même valeur absolue d'amplitude/tension faisant que la tension moyenne appliquée entre l'électrode d'excitation et l'électrode de référence est nulle (une seule paire de ces électrodes étant excitée lors d'une mesure).

En dehors des mesures/acquisitions, le pont-en-H est mis en configuration ouverte (i.e. sortie flottante) ou son alimentation est coupée. Il en résulte qu'aucune tension n'est présente sur sa sortie. En alternative ou complément on prévoit de désélectionner le démultiplexeur, i.e. rendu non conducteur ses ports d'entrées-sortie et ses ports d'entrées étant flottants, afin qu'aucun courant ne puisse circuler à travers les électrodes et l'eau.

Le pont-en H est contrôlé par un microcontrôleur à programme de contrôle de l'ensemble électronique du système. Ce microcontrôleur à programme de contrôle est configuré pour que la fréquence d'excitation pour la tension alternative produite en sortie par le pont-en H soit variable en fonction de la plage de conductivité à mesurer. De préférence, le microcontrôleur à programme de contrôle adapte la fréquence d'excitation pour la tension alternative en fonction de premières mesures de conductivités . Si la fréquence d'excitation utilisée est correcte pour la/les mesures, la fréquence reste inchangée, sinon elle est modifiée. Cette modification est effectuée de manière à ce que la fréquence d'excitation augmente avec la conductivité.

A titre d'exemple de fréquences définies en fonction des plages de mesure, on propose :

- pour une conductivité ($\mu$S/cm) entre 0 et 1999, une fréquence d'excitation de 500 Hz
- pour une conductivité ($\mu$S/cm) entre 2000 et 9999, une fréquence d'excitation de 1000 Hz
- pour une conductivité ($\mu$S/cm) de 10000 et plus, une fréquence d'excitation de 5000 Hz

Matériellement, le système de l'invention comporte plusieurs éléments : un capteur et un ensemble électronique.

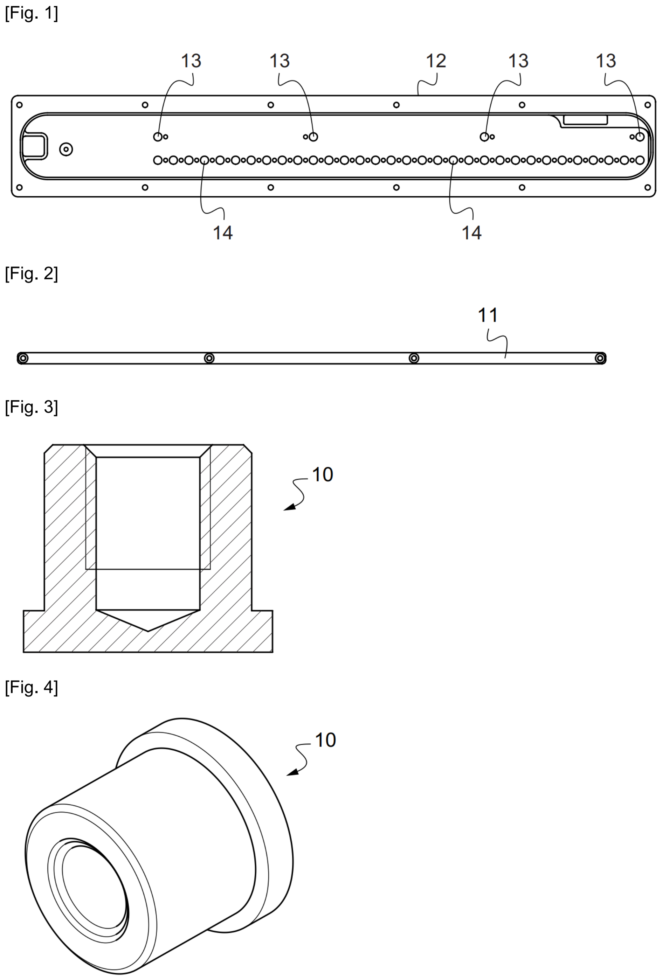

Figures 1 à 4 et 7, le capteur 1 est constitué d'un boîtier 12 allongé en hauteur et en surface duquel les électrodes d'excitation 10 et de référence 11 sont agencées. Le capteur 1 comporte une électrode de référence 11 et un certain nombre d'électrodes d'excitation 10, en général multiple de 8, par exemple 16, 32 voire 64 électrodes d'excitation 10. Les électrodes d'excitation 10 sont circulaires de diamètre 5 mm et séparées entre elles de 5 mm, les centres de deux électrodes d'excitation successives étant séparés de 10 mm. L'électrode de référence 11 est allongée verticalement sur la hauteur du capteur 1. Les extrémités inférieure et supérieure de l'électrode de référence sont de préférence arrondies. La largeur de l'électrode de référence est sensiblement constante sur sa hauteur. Les électrodes d'excitation 10 apparaissent sensiblement ponctuelles par rapport à l'électrode de référence 11. Les électrodes d'excitation 10 sont alignées verticalement parallèlement à l'électrode de référence 11 et à une distance constante de cette dernière.

Figure 1, le boîtier 12 est représenté seul et il comporte en surface des orifices 13, 14 traversant l'épaisseur de la paroi pour fixation des électrodes sur le boîtier.

Figure 2, l'électrode de référence 11 est représentée seule et elle comporte des pions de fixation au boîtier 12 et destinés à venir se placer dans les orifices 13 pour fixation de l'électrode de référence au boîtier et aussi raccordement électrique de cette dernière à l'intérieur du boîtier.

Figures 3 et 4, une des électrodes d'excitation 10 est représentée seule et elle est en forme approximative de champignon et est destinée à venir se placer dans un des orifices 14 pour fixation de l'électrode d'excitation au boîtier 12 et aussi raccordement électrique de cette dernière à l'intérieur du boîtier.

On comprend que les électrodes 10, 11 sont affleurantes à la surface du boîtier 12. Les fixations et raccordements électriques s'effectuent par vissage de vis dans les pions alésés et taraudés de l'électrode de référence 11 (ou boulonnage si les pions sont filetés) et dans les alésages taraudés des électrodes d'excitation 10. Une fois les électrodes 10,11 installées, fixées et raccordées électriquement aux autres éléments disposés dans le boîtier 12, ce dernier est finalement fermé/clos d'une manière étanche. Avantageusement le contenu du boîtier peut être rempli d'un matériau de remplissage polymérisable par exemple de type élastomère.

Le capteur 1 qui est disposé au contact de l'eau du réseau d'assainissement comporte donc en surface des électrodes 10, 11 qui sont ou non immergées au contact de ladite eau en fonction du niveau/de la hauteur de ladite eau le long de la hauteur du capteur.

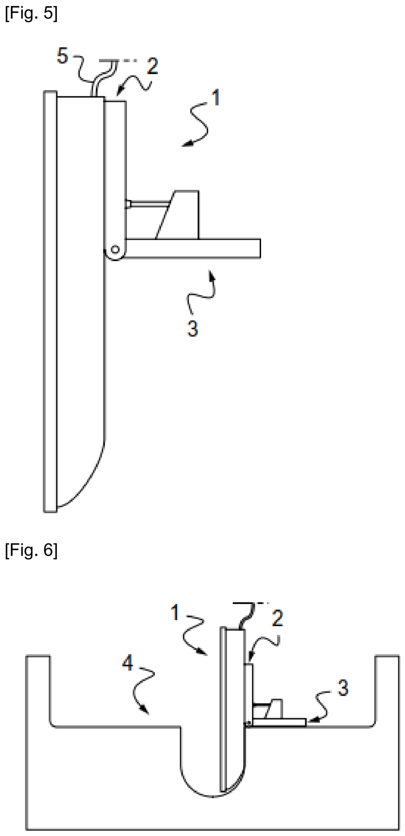

Dans une application à une eau d'un réseau d'assainissement circulant dans une cunette 4, figures 5 et 6, le capteur 1 est avantageusement fixé sur un support adaptateur 2 comportant un socle 3 orientable fixé sur un rebord de la cunette 4. Le socle 3 orientable permet de régler la disposition du capteur 1 afin qu'il soit positionné verticalement et que l'électrode de référence 11 soit verticale (et donc l'alignement des électrodes d'excitation 10 aussi). Le support adaptateur 2 peut également comporter un moyen de réglage de la position en hauteur du capteur 1 par rapport au socle 3, e.g. par coulissement, afin de pouvoir plonger/descendre plus ou moins le capteur dans la cunette 4. Avantageusement, le boîtier du capteur est d'épaisseur réduite afin de ne pas perturber l'écoulement de l'eau dans les applications mesurant le débit.

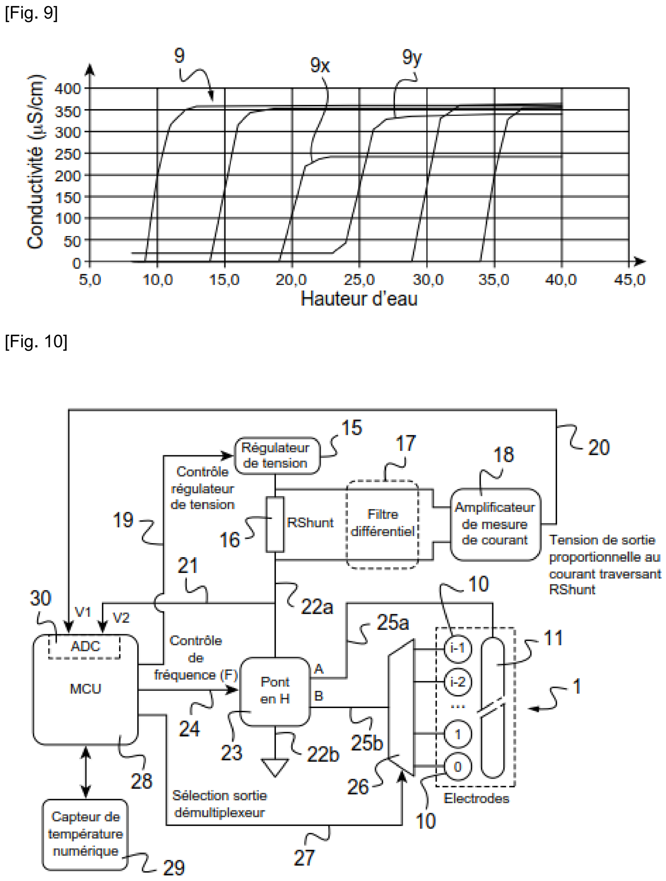

L'ensemble électronique figure 10 comporte plusieurs circuits électroniques qui sont de préférence agencés en des emplacements différents.

Ces circuits électroniques sont au moins un circuit générateur de tension constante 15, un pont-en-H 23, un circuit de mesure de courant d'alimentation de pont 16, 17, 18, 20, un circuit de mesure de tension d'alimentation de pont 21, un démultiplexeur 26 analogique, un microcontrôleur à programme de contrôle 28, au moins un circuit de mémoire numérique relié ou interne au microcontrôleur, dont mémoire persistante, un circuit de mesure de température 29 de l'eau du réseau d'assainissement. D'autres circuits électroniques peuvent être prévus, par exemple un circuit d'interface de communication, filaire (e.g. communication Modbus RS485) ou non, pour des échanges de données (e.g. mesures) et/ou programmes avec l'extérieur du système. Par exemple un circuit d'interface d'affichage (e.g. affichage de mesures), un circuit d'interface d'entrée (e.g. un clavier étanche).

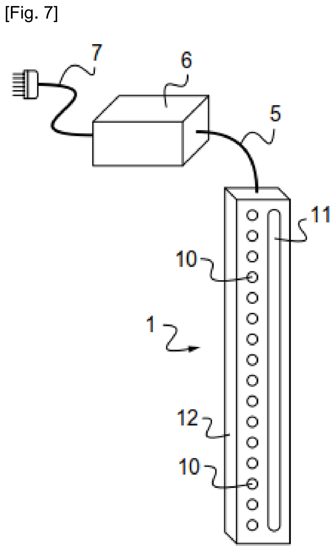

De préférence, comme dans la mise en œuvre représentée figure 7, le démultiplexeur 26 analogique et le circuit de mesure de température 29 de l'eau du réseau d'assainissement sont dans le boîtier 12 du capteur 1, le reste des circuits électroniques étant dans un autre boîtier, dit boîtier de contrôle 6, à distance du boîtier du capteur, les deux étant reliés par une liaison filaire 5 (e.g. protocole I2C). Le boîtier de contrôle 6 comporte une liaison filaire 7 pour communication, avantageusement communication Modbus RS485, avec l'extérieur du système. On comprend que d'autres moyens de communication peuvent être mis en œuvre dans d'autres modes de réalisation.

Le Modbus met en œuvre des registres et avantageusement certains de ces registres permettent, s'ils sont appelés, de déclencher une action, par exemple, une mesure, une remise à zéro du système, un étalonnage du système.

En effet, le démultiplexeur 26 est chargé du démultiplexage du signal d'excitation 25A, 25B sortant du pont-en-H 23 pour sélectionner/connecter une à la fois des électrodes d'excitation 10. Or le capteur 1 peut comporter 16 électrodes d'excitation, voire plus : 32 ou 64, et il est préférable de réduire au maximum la longueur des liaisons entre le démultiplexeur 26 et les électrodes d'excitation 10 et aussi d'éviter une liaison filaire 5 avec autant de fils.

Un tel agencement en un boîtier 12 de capteur 1 et un boîtier de contrôle 6 comportant le microcontrôleurs, laisse toute attitude pour choisir l'emplacement du boîtier de contrôle 6 qui peut être ainsi placé en un endroit plus sûr et accessible si nécessaire.

Les microcontrôleurs 28 avantageusement utilisés dans le système, présentent l'avantage de comporter outre un microprocesseur, des circuits d'interface, notamment un/des convertisseurs analogiques-numériques (ADC) 30, et de la mémoire, e.g. mémoire EEPROM/« Flash », i.e. persistante, et RAM, et sont possiblement reprogrammables. Toutefois, on prévoit de mettre en œuvre une mémoire persistante distincte reliée au microcontrôleur pour mémoriser des données. En particulier les données concernant le capteur peuvent être mémorisées dans une mémoire persistante dans le boîtier du capteur, notamment données de configuration du capteur (e.g. nombre d'électrodes d'excitation), données d'étalonnage propres aux électrodes, e.g. les constantes de cellules $K_i$ des différentes électrodes d'excitation. On peut aussi avantageusement mémoriser dans le système, notamment dans le microcontrôleur, des données propres au système et/ou au capteur, e.g. la valeur de décalage Z de mesure de courant permettant d'effectuer par calcul une mise à zéro des courants de fuite, plus précisément permettant de supprimer/corriger les effets des courants de fuite dans les mesures. Ces microcontrôleurs utilisables peuvent également comporter un/des convertisseurs analogique-numérique (i.e. ADC « Analog to Digital Converter »), un/des convertisseurs numérique-analogique (i.e. DAC "Digital to Analog Converter"), des moyens de communication dont notamment SPI (i.e. « Serial Peripheral Interface ») et/ou CAN (i.e. « Controller Area Network »), une horloge temps réel (i.e. RTC "Real-Time Clock").

Ainsi, dans une modalité de réalisation particulièrement avantageuse, le boîtier 12 du capteur 1 comporte une mémoire persistante stockant des données de configuration propres au capteur, par exemple le nombre d'électrodes d'excitation, de préférence les constantes de cellules $K_i$ des différentes électrodes d'excitation et/ou toute autre donnée spécifique du capteur en question. Cette mémoire persistante dans le capteur peut possiblement comporter en outre le numéro de série du capteur, sa date de fabrication et, éventuellement, le/les numéros de versions des programmes du microcontrôleur pouvant utiliser le capteur en question. Dans une modalité de réalisation, la mémoire persistante du système (dont celle du capteur) peut servir à stocker des informations sur des évènements (e.g. pannes, réparations) ou actions s'étant produits.

Ainsi, il est possible de changer un capteur dans un système sans avoir à recalibrer/réétalonner tout le système, le microcontrôleurs 28 à programme de contrôle du boîtier de contrôle 6 étant configuré pour s'adapter à différents capteurs en utilisant les données de configuration propre de chaque capteur contenues dans la mémoire persistante du capteur 1.

A titre d'exemple de microcontrôleur, on peut utiliser le STM32L431RCT6 qui dispose d'une mémoire persistante « Flash » de 256K-bit et une RAM de 64K-bit. De préférence, les alimentations et/ou activations des circuits électroniques de l'ensemble électronique sont sous le contrôle du microcontrôleur afin de pouvoir réduire au maximum la consommation du système en dehors des moments de mesures, le microcontrôleur pouvant lui-même passer dans un mode basse consommation.

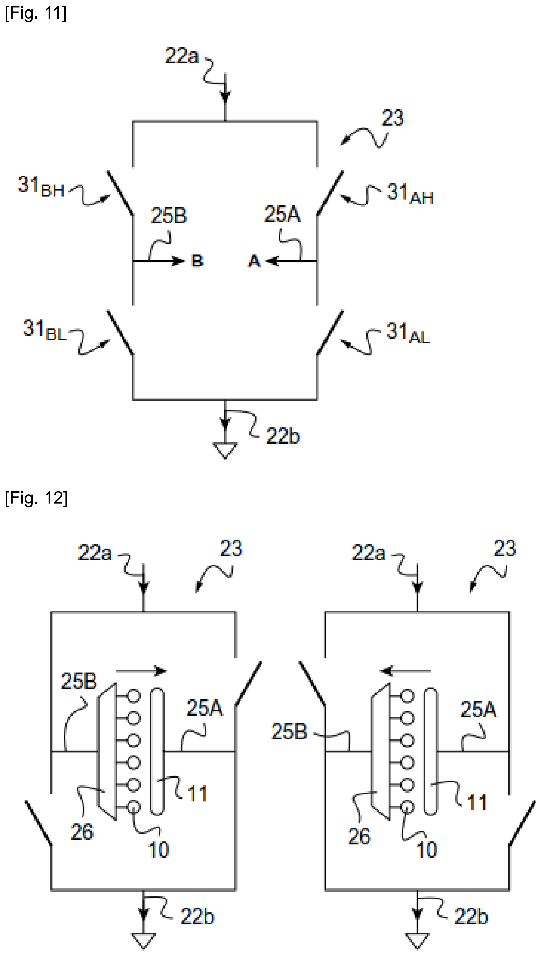

Pour la génération de la tension alternative envoyée sur les électrodes pendant la mesure, un pont-en-H 23 est donc utilisé (figures 11, 12). Ce pont-en-H 23 comporte une entrée d'alimentation ayant deux connexions d'alimentation 22a et 22b recevant une tension d'alimentation de pont. Il comporte également une sortie de pont ayant deux connexions de sortie (réf. A) 25A et (réf. B) 25B produisant entre elles une tension de sortie de pont. Il comporte également une entrée de commande de pont 24 permettant de configurer chacun des semiconducteurs des deux push-pull (i.e. montage symétrique) qu'il comporte en passant ou non-passant. L'entrée de commande de pont 24, permet d'obtenir, selon les commandes de pont 24, une coupure de la tension sur la sortie de pont (sortie de pont A/25A et B/25B) ; des inversions de tension sur la sortie de pont.

Le pont-en-H 23 comporte un premier push-pull avec deux interrupteur commandés 31BH et 31BL et point commun/milieu de sortie B, typiquement des transistor en technologie MOS, agencé entre les deux connexions d'alimentation 22a et 22b. Le pont-en-H 23 comporte aussi un second push-pull avec deux interrupteur commandés 31AH et 31AL et point commun/milieu de sortie A, typiquement des transistor en technologie MOS, agencé entre les deux connexions d'alimentation 22a et 22b.

Selon les transistors rendus passants, on envoi sur la sortie de pont A, B la tension d'alimentation de pont (22a-22b) inversée ou non (fig. 12) et il en résulte que le courant circulant (par 25A, 25B) dans les électrodes s'inverse alternativement. L'électrode de référence 11 est relié à la connexions de sortie A (par 25A) du pont-en-H 23.

Le démultiplexeur 26 analogique permet de sélectionner une électrode d'excitation 10 à la fois pour qu'elle émette ou reçoive le courant circulant dans les électrodes (i.e. une électrode d'excitation et l'électrode de référence) du fait de l'application de la tension de sortie de pont (par 25A, 25B). Le démultiplexeur 26 comporte un port de sortie analogique relié à la connexions de sortie B (par 25B) du pont-en-H 23. Le démultiplexeur 26 comporte un ensemble de ports d'entrées analogiques chacun relié à une électrode d'excitation 10 correspondante. Le démultiplexeur 26 comporte aussi une entrée de commande de sélection d'entrée permettant en fonction de la commande de sélection d'entrée (par 27) de relier ou non, un seul à la fois, le port d'entrée sélectionné au port de sortie. Pour cela, les ports d'entrée non sélectionnés sont rendus flottants (i.e. haute impédance). Dans une modalité de réalisation, l'entrée de commande de sélection d'entrée peut permettre de désactiver le démultiplexeur, i.e. couper toute liaison électrique entre le port de sortie et les ports d'entrée par exemple par mise en haute impédance de tous les ports de sortie. Pour le pont-en-H 23, il est possible d'utiliser un composant électronique normalement utilisé pour la commande/contrôle de moteurs courant continu (DC). En effet, ce type de composant permet de contrôler la direction de rotation d'un moteur DC (i.e. courant continu) en inversant le sens du courant et aussi de contrôler sa vitesse de rotation grâce à des signaux modulés en largeur d'impulsion (PWM). Cette dernière possibilité permet de contrôler la fréquence d'excitation du signal carré produit en sortie du pont-en-H.

Le circuit générateur de tension constante 15 est un simple régulateur de tension qui est contrôlé (par 19) par le microcontrôleur 28. Ce contrôle est tout ou rien ou dans une modalité plus avancée, peut agir sur la valeur de la tension constante générée, par exemple en pouvant la faire varier entre 2 V et 5 V. Dans l'exemple décrit le circuit générateur de tension constante 15 est contrôlé par le microcontrôleur en tout ou rien (par coupure ou connexion de son alimentation) et est configuré pour générer une tension fixe de 3V. Son impédance de sortie est suffisamment faible pour considérer que le système fonctionne à tension constante pour les courants à générer dans le circuit des électrodes.

Le circuit générateur de tension constante 15 fournit la tension d'alimentation de pont (en 22a) par l'intermédiaire du circuit de mesure de courant d'alimentation de pont 16, 17, 18, 20.

Le circuit de mesure de courant d'alimentation de pont permet de mesurer le courant destiné à l'entrée d'alimentation du pont-en-H. Ce courant d'alimentation entrant (par 22a) dans le pont-en-H 23 est sensiblement égal au courant passant par la sortie de pont (par A/25A et B/25B) et donc au courant passant par les électrodes 10, 11. Toutefois, le pont-en-H 23 peut lui-même consommer une partie du courant l'alimentant et même si cette consommation propre est faible et pourrait être considérée négligeable, il est préférable d'en tenir compte et de la retirer de la mesure de courant d'alimentation de pont servant au calcul de la conductance locale et/ou de la conductivité locale. A cette fin il est mis en œuvre une correction dans la mesure du courant en utilisant une valeur de décalage Z de mesure de courant permettant d'effectuer par calcul une mise à zéro des courants de fuite pouvant exister au sein du système (plus précisément permettant de supprimer/corriger les effets des courants de fuite dans les mesures).

La détermination de la donnée de mise à zéro Z est basée sur le fait qu'il faut que la mesure de courant retourne zéro lorsque qu'aucun courant ne passe par les électrodes, par exemple parce que le capteur est au sec ou que le démultiplexeur est désactivé et/ou que la sortie de pont est flottante (aucun des transistors du pont-en-H n'est passant ou conducteur) ou que, moins préférablement, les deux sorties A et B sont mises à la masse ou au potentiel d'alimentation (tension en 22a). En pratique et plus simplement, la détermination de la donnée de mise à zéro Z pendant la phase d'étalonnage s'effectue par une mise à sec du capteur.

Le circuit de mesure de courant d'alimentation de pont met en œuvre une résistance de shunt 16 et la chute de tension aux bornes du shunt 16 est mesurée par un amplificateur différentiel 18 dont la tension de sortie V1 (sur la connexion 20) est proportionnelle au courant circulant dans le shunt 16. De préférence la valeur du shunt est la plus faible possible et on peut par exemple prendre un shunt de $0,5\Omega \pm 1\%$, 1W. Il est avantageusement mis en œuvre un filtre différentiel 17 à l'entrée de l'amplificateur différentiel. Etant donné la fréquence d'excitation du signal produit par le pont-en-H, entre 500Hz et 5000Hz, le filtre passe-bas différentiel disposé entre les bornes de la résistance de Shunt a une fréquence de coupure de 800Hz afin d'éliminer les éventuelles perturbations liées à la génération du signal alternatif carré par le pont-en-H. La connexion 20 transmet la tension V1 à un convertisseur analogique-numérique (ADC) 30 du microcontrôleur 28 afin que ce dernier puisse acquérir numériquement la valeur du courant mesuré.

Le circuit de mesure de tension d'alimentation de pont 21 mesurant la tension d'alimentation de pont se résume à une connexion 21 à la ligne 22a (i.e. en aval du shunt 16, le circuit générateur de tension constante 15 étant considéré en amont du shunt) servant à l'alimentation du pont-en-H 23. La connexion 21 transmet la tension V2 correspondant à la tension d'alimentation du pont-en-H 23à un convertisseur analogique-numérique (ADC) 30 du microcontrôleur 28 afin que ce dernier puisse acquérir numériquement la valeur de la tension. Un filtrage peut être mis en œuvre sur la connexion 21.

On peut prévoir des composants électroniques de protection contre des surtensions sur les lignes électriques sortant du pont-en-H et menant aux électrodes.

Le système de l'invention peut s'appliquer à la réalisation d'un « data logger », c'est-à-dire d'un enregistreur/appareil de collecte de données provenant du capteur. Le système comporte alors une quantité de mémoire persistante suffisante pour collecter au moins les mesures résultant des calculs effectués et les références temporelles correspondantes (i.e. le moment de la mesure). A cette fin un circuit de mémoire persistante spécifique peut être mis en œuvre, par exemple sous forme d'une carte mémoire amovible. Il est aussi possible de décharger les données collectées par la liaison filaire 7 de communication Modbus. Dans une variante une communication sans fil peut être mise en œuvre.

Dans un mode de réalisation particulier l'ensemble électronique comporte en outre un circuit électronique d'horloge temps réel (i.e. date + temps) si le microcontrôleur n'en comporte pas ou ne peut pas se charger d'en simuler une.

Le programme exécuté par le microcontrôleur peut avantageusement permettre l'optimisation de la consommation du système, la mise en œuvre de nombreux protocoles de communication radio et/ou filaire. Le programme est de préférence configuré pour effectuer des mesures et possiblement les collecter avec de nombreux types de capteurs du fait que les capteurs comportent avantageusement une mémoire persistante contenant leurs caractéristiques et que le programme du microcontrôleur permet d'adapter les mesures, dont les calculs, au capteur utilisé. Le programme est avantageusement configuré avec des librairies de bas niveau spécifiques fournies par le constructeur du microcontrôleur qui constituent une première couche d'abstraction matérielle, et des librairies de haut niveau pour les librairies de composants et de la couche applicative. Il est aussi avantageusement mis en œuvre pour le programme, une couche dite « Board Support Package » qui décrit les périphériques internes et externes spécifiques à l'ensemble électronique afin de pouvoir utiliser le système avec divers périphériques, les périphériques utilisés y étant décrit pour que le programme s'adapte à ceux-ci.

Le déroulement d'une mesure avec le système est maintenant décrit.

Les mesures peuvent être déclenchées de deux manières différentes :

-Automatiquement à la mise sous tension du capteur puis sur demande de l'utilisateur,
- Uniquement sur demande de l'utilisateur.

L'utilisateur peut déclencher une mesure en écrivant une valeur quelconque dans un registre Modbus spécifique. Lors du déclenchement d'une mesure, les étapes suivantes s'exécutent :

1. La fréquence d'excitation est déterminée en fonction de la dernière valeur de conductivité locale ou globale mesurée. Si aucune mesure n'a été effectuée avant celle en cours, une fréquence d'excitation par défaut est utilisé. Une variable compteur « Num, Electrode » de sélection de paire d'électrodes est mise à zéro.

2. La fréquence d'excitation sélectionnée est appliquée au composant pont en H sous forme d'un signal PWM permettant de produire en sortie de pont un signal carré périodique.

3. La sortie de démultiplexeur correspondant à l'électrode d'excitation de numéro/indice « Num, Electrode » est sélectionnée afin que les sorties du pont-en-H soient électriquement reliées à la paire d'électrodes « Num, Electrode » par l'intermédiaire du démultiplexeur. Pour « Num, Electrode » = 0 c'est la paire d'électrodes la plus basse du capteur qui est sélectionnée.

4. Le régulateur de tension est allumé et génère une tension de 3VCC à l'entrée du pont-en-H.

5. Un délai de 5ms est appliqué afin de permettre au régulateur de monter en tension.

6. Les tensions V1 et V2 sont mesurée par le convertisseur analogique- numérique du microcontrôleur. Ces mesures sont itérées un nombre déterminé de fois déterminé par l'utilisateur (de 5 à 25 au choix par incrément de 5) en fonction de son besoin en précision. Les valeurs de tensions V1 et V2 utilisées pour les calculs ultérieurs sont alors des moyennes sur ces mesures itérées.

7. Le régulateur de tension est éteint.

8. Le courant électrique local $I_i$ est calculé à partir de la valeur moyennée de V1. La tension locale $V_i$ étant égale à la valeur moyennée de V2. L'indice i étant égal à la valeur de la variable « Num, Electrode ».

9. La conductance locale de l'eau du réseau d'assainissement est calculée avec une correction par $Z$ des courants de fuite, la correction étant appliquée à $I_i$. Le calcul de la conductivité locale $EC_{25i}$ ($i$ étant égal à la valeur de la variable « Num, Electrode ») est également effectué, la température de l'eau ayant été mesurée initialement, avant le début des étapes. Chaque conductivité locale $EC_{25i}$ est mémorisée. En variante, la conductivité locale $EC_{25i}$ est directement calculée à partir de $I_i$ et $V_i$.

10. Les étapes 3 à 9 se répètent en effectuant chaque fois la mesure sur l'électrode suivante en incrémentant de 1 la valeur de la variable « Num, Electrode » : « Num, Electrode » = « Num, Electrode » +1 ;

et jusqu'à ce que toutes les électrodes aient été mesurées OU qu'un nombre de paires d'électrodes (paramètre Modbus STOP_AFTER) prédéterminé par l'utilisateur produisent une valeur de conductivité locale $EC_{25i}$ nulle, indiquant qu'elles sont probablement hors de l'eau ou défaillantes. Ce paramètre Modbus STOP_AFTER est par défaut égal à 2. Il peut être modifié en particulier si on sait qu'il existe des électrodes défaillantes. Par exemple ce paramètre peut être augmenté par multiples de 2.

On comprend donc que seule la paire d'électrodes subissant les mesures est alimentée par le pont-en-H et que les mesures s'effectuent sur une seule paire d'électrodes à la fois.

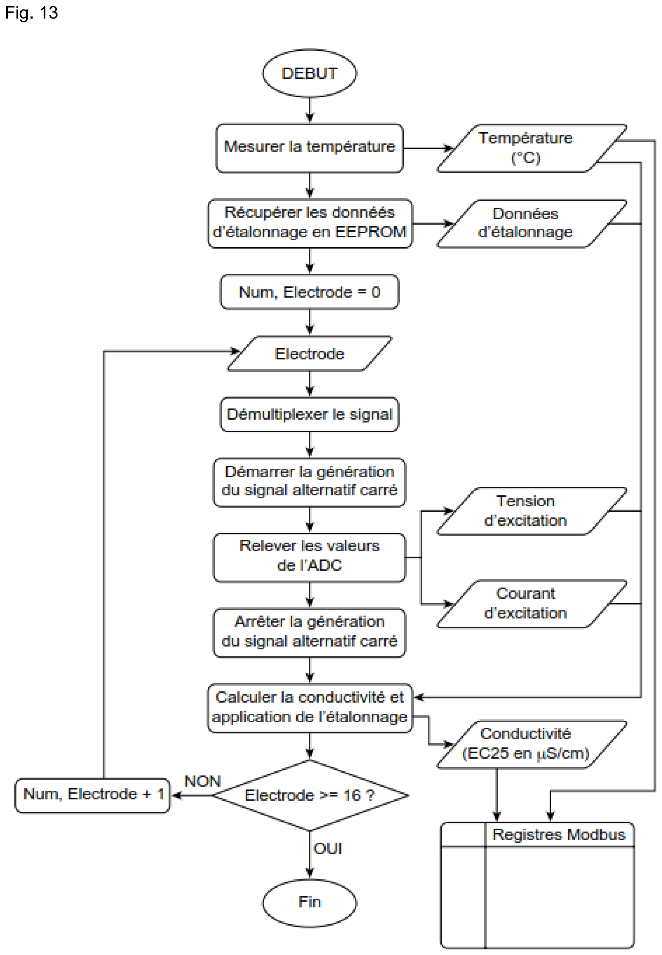

Plus précisément, il est présenté en relation la figure 13, un diagramme de flux dans le cas où la mesure à obtenir est la conductivité globale $EC_{25}$ corrigée de la température pour un capteur de 16 paires d'électrodes.

Le processus de mesure commence au début par une mesure de la température qui est mémorisée puis les données d'étalonnage (e.g. constantes de cellules $K_i$) sont récupérées de la mémoire persistante (de préférence mémoire persistante dans le capteur) puis un compteur de numéro/indice d'électrode avec la variable « Num, Electrode » est mis à zéro (i.e. numéro de sélection de l'électrode d'excitation du bas du capteur). Ensuite le démultiplexeur sélectionne l'électrode d'excitation par le numéro d'électrode dans le compteur, puis le pont-en-H est commandé afin de démarrer la génération du signal alternatif carré et pendant cette génération des acquisitions de mesures de la tension d'excitation (i.e. tension d'alimentation du pont-en-H) et du courant d'excitation (i.e. courant d'alimentation du pont-en-H) sont effectuées afin de relever les valeurs du convertisseur analogique-numérique. Après ces acquisitions, la génération du signal alternatif carré est arrêtée et la conductivité locale est calculée en appliquant les données d'étalonnage ($Z$ et $K_i$ pour l'électrode sélectionnée par le compteur) et la conductivité locale est stockée dans un des registres Modbus. Ces opérations sont répétées après incrémentation du compteur de numéro d'électrode afin d'effectuer des mesures sur toutes les électrodes d'excitation en remontant du bas du capteur vers le haut, un test du numéro d'électrode permettant d'arrêter le processus une fois toutes les électrodes mesurées, i.e. arrêt si « Num, Electrode » >= $N$ avec $N$ égal à 16 dans cet exemple. On comprend qu'il est possible d'ajouter des tests supplémentaires, par exemple pour arrêter le balayage si deux mesures successives de conductivité locale sont nulles alors que les précédentes ne l'étaient pas.

D'une manière générale, les mesures peuvent être déclenchées de deux manières différentes :

- Automatiquement à la mise sous tension du système puis sur demande d'un utilisateur, ou
- Uniquement sur demande d'un utilisateur. En effet, le système est configuré comme un automate programmable et met en œuvre un protocole de communication Modbus en mode RTU, ici RS485, et l'utilisateur qui connecté au système peut déclencher une activité, en l'espèce une mesure, en écrivant dans un registre Modbus spécifique. On comprend que d'autres moyens et protocoles de communication peuvent être mis en œuvre dans d'autres modes de réalisation. Dans le cas d'une application où le système est utilisé en enregistreur/appareil de collecte de données (i.e. « data logger »), les mesures s'effectuent automatiquement selon une programmation prédéfinie, par exemple périodique ou sous condition (e.g. variation de température, détection spécifique...).

On considère qu'étant donné que le processus de mesure est rapide, la température de l'eau du réseau d'assainissement ne change pas significativement au cours de la mesure et, en conséquence, la température n'est mesurée qu'une seule fois (des itérations pouvant toutefois être mise en œuvre au cours de cette seule mesure afin d'effectuer un moyennage pour augmenter la précision de la mesure de température) au tout début du processus. La valeur de température obtenue est gardée en mémoire, typiquement RAM, pour les besoins des calculs des conductivités locales corrigées des effets de la température. La valeur de température obtenue est également stockée dans un registre Modbus afin qu'elle soit accessible aux utilisateurs. Dans le cas où le système est utilisé en enregistreur/appareil de collecte de données (i.e. « data logger ») la valeur de température peut également être associée aux mesures enregistrées/collectées. Afin de pouvoir effectuer la mesure, il est aussi nécessaire de récupérer les données d'étalonnage, i.e. les constantes de cellule $K_i$ et la valeur de décalage Z de correction de mesure de courant, qui sont dans la mémoire persistante du capteur si on souhaite des mesures précises et les données de configuration du capteur, en particulier le nombre d'électrodes d'excitation et/ou de ports d'entrée du démultiplexeur. A noter que cette dernière information peut éventuellement être obtenue indirectement par le nombre de constantes de cellules $K_i$ qui sont chacune spécifique d'une électrode d'excitation donnée.

Pour la suite des explications sur l'exemple décrit, il est considéré par convention que les électrodes d'excitation (et donc les paires d'électrodes correspondantes) sont référencées/numérotées de 0 à 15 du bas du capteur vers le haut du capteur qui comporte 16 paires d'électrodes. Du fait de la configuration de l'ensemble électronique, les mesures ne peuvent être effectuées qu'avec une électrode d'excitation alimentée à la fois et il est donc nécessaire de balayer l'ensemble des électrodes d'excitation pour obtenir les mesures pour chacune d'entre elles.

De préférence, comme représenté, la première paire d'électrodes qui est mesurée est la numéro 0, i.e. l'électrode d'excitation la plus basse. Ainsi, lorsque qu'au cours du balayage en remontant dans les différentes électrodes successivement, une mesure de conductivité locale est nulle, on peut considérer que le niveau/plan supérieur de l'eau du réseau d'assainissement est juste en dessous de celle à conductivité locale nulle si les précédentes électrodes d'excitation avaient une conductivité locale non-nulle et on peut alors envisager d'arrêter le balayage. Toutefois, par mesure de sécurité, il est préférable de continuer le balayage au cas où la conductivité locale nulle serait dû à une anomalie (e.g. électrode défaillante, défaut du circuit électronique et/ou du câblage) car dans ce cas il y aura au moins une paire d'électrodes supérieure à conductivité locale non nulle. On peut, à titre intermédiaire, effectuer une seule mesure supplémentaire sur l'électrode d'excitation suivante, i.e. juste au-dessus, de celle à mesure nulle qui donnera une mesure de conductivité locale nulle (rendant plus probable que le niveau/plan supérieur de l'eau du réseau d'assainissement est effectivement plus bas) ou donnera une mesure de conductivité locale non-nulle (qui rendra probable qu'il existe une anomalie avec l'électrode d'excitation à mesure de conductivité locale nulle).

Un compteur d'électrode pour le choix du port d'entrée du démultiplexeur 26 est initialisé à 0, ce compteur servant au comptage du balayage des différentes électrodes d'excitation. La valeur du compteur d'électrode est transmise à la fonction du programme responsable du démultiplexage.

Une fois que l'électrode d'excitation sélectionnée est connectée par le démultiplexeur à la sortie du pont-en-H 23, la génération du signal alternatif carré est démarrée par commande (par 24) du pont-en-H 23 qui est alimenté par une tension continue de typiquement de 3V fournie par le circuit générateur de tension constante 15. Le circuit générateur de tension constante 15 recevant avantageusement son alimentation par l'intermédiaire d'un interrupteur, e.g. MOSFET coupe circuit sur alimentation, afin de limiter la consommation du système en dehors des mesures, on comprend que cet interrupteur est rendu passant pour pouvoir fournir ces 3V typiques. De préférence, la commande (par 24) du pont-en-H 23 pour génération du signal alternatif carré est commencée un peu avant que le circuit générateur de tension constante 15 ne fournisse les 3V typiques. A noter que si la sortie du pont-en-H 23 peut être rendue flottante (i.e. aucun courant ne circulant par les électrodes) indépendamment de la commande (par 24) du pont-en-H 23 pour génération du signal alternatif carré alors l'ordre d'application des alimentations aura moins d'importance et on laissera flottante la sortie jusqu'à ce que les activations des alimentations et la commandes de pont pour génération du signal alternatif carré aient été faites.

Après un court temps de stabilisation, par exemple de 5ms pour une fréquence d'excitation de 500Hz, le courant et la tension issus respectivement du circuit de mesure de courant d'alimentation de pont 16, 17, 18, 20, et du circuit de mesure de tension d'alimentation de pont 21, sont acquises par le convertisseur analogique-numérique du microcontrôleur.

Ces mesures et acquisitions sont itérées pour chaque paire d'électrodes afin d'effectuer un moyennage améliorant la précision. Ainsi, il est effectué des moyennes sur cinq acquisitions pour le courant et la tension d'excitation. Ces itérations correspondent à un nombre n de mesures, e.g. de 5 à 25 par incrément de 5, déterminé par l'utilisateur en fonction de son besoin en précision.

Le courant mesuré est obtenu à partir de la valeur acquise (moyennée) de V1 (figure 10) via la connexion 20.

La tension mesurée est obtenue à partir de la valeur acquise (moyennée) de V2 via la connexion 21.

La conductivité locale (i.e. conductivité pour l'électrode d'excitation sélectionnée) est calculée en mettant en œuvre une correction des courants de fuite grâce à la valeur de décalage Z de mesure de courant, une correction avec la constante de cellule $K_i$ et une prise en compte des effets de la température. Le calcul est le suivant :

$$EC_{25i} = \frac{CO_i * K_i}{1 + ((T° - 25) * 0,02)}$$

avec $Co_i = (I_i - Z) / V_i$ la conductance locale corrigée des courants de fuite pour la paire d'électrodes d'indice $i$, $EC_{25i}$ la conductivité locale ramenée à 25°C en S/cm pour la paire d'électrodes d'indice $i$ ; $K_i$ la constante de cellule de la paire d'électrodes d'indice $i$, T° la température mesurée en °C de l'eau du réseau d'assainissement.

Ce/ces calculs de conductivité locale $EC_{25i}$ peuvent être effectués avant de passer à la mesure de l'électrode d'excitation suivante ou, alors, après la fin des mesures de courants et tensions locales (i.e. après la dernière paire d'électrodes mesurée). Une fois les acquisitions du courant et de la tension effectuées, l'alimentation du circuit générateur de tension constante 15 est coupée et la commande de pont pour génération du signal alternatif carré arrêtée.

Ces mêmes opérations sont répétées pour balayer chacune des paires d'électrodes jusqu'à, de préférence, la rencontre d'une électrode présentant une conductivité locale nulle (ou un autre critère comme expliqué ci-dessus en considération de la possibilité d'une anomalie).

Pour chaque électrode d'excitation sélectionnée et acquisitions du courant et tension puis moyennage, les calculs des conductivités locales sont effectués selon les formules présentées dans le cadre de la présente invention.

Les valeurs de conductivités locales ainsi obtenues sont stockées dans de la mémoire persistante du système et de préférence dans des registres Modbus.

De préférence, la fréquence d'excitation pour basculement de la tension de sortie du pont-en-H destiné aux inversions de sens du courant d'excitation des électrodes est déterminée en fonction de la dernière valeur de conductivité locale mesurée. Si aucune mesure n'a été effectuée avant celle débutée, une fréquence d'excitation par défaut est utilisée

La fréquence d'excitation sélectionnée est appliquée au circuit pont-en-H sous forme d'une commande de pont permettant de générer en sortie du pont-en-H la tension alternative carrée décrite précédemment. Selon le composant électronique qui est utilisé pour le pont-en-H, cette commande de pont peut être un signal PWM ou un signal carré.

Les valeurs de conductivités locales $EC_{25i}$ mémorisées pour chaque électrode d'excitation du capteur peuvent ensuite servir au calcul de la conductivité globale $EC_{25}$, au calcul de la salinité, au calcul du niveau de l'eau, le calcul de la hauteur de sédiments, la détection d'électrodes défectueuses ou défaillante et du type d'encrassement...

Les valeurs de conductivités locales $EC_{25i}$ peuvent aussi être mémorisées pour différents moments, e.g. quatre fois par jour, pour permettre de déterminer les évolutions de l'eau et par exemple sa composition.

Concernant le calcul du niveau de l'eau, il est possible d'utiliser le système pour déterminer le niveau relatif de l'eau par rapport au capteur.

Dans le cas du capteur dont les électrodes d'excitation sont espacées centre à centre de 10mm (i.e. le pas e d'espacement entre deux électrodes d'excitation successives le long de la hauteur du capteur est égal à 10 mm), il est possible de déterminer le niveau de l'eau avec une précision sensiblement égale à 10 mm si considère le tout-ou-rien, i.e. passage d'une mesure d'une conductivité locale non-nulle à une conductivité locale nulle pour la paire d'électrode suivante vers le haut. Toutefois, grâce à l'invention, il est possible de mesurer précisément la conductivité locale $EC_{25i}$ et donc de suivre l'évolution de ces valeurs et donc des variations millimétriques du niveau de l'eau.

A cette fin, on propose de calculer la hauteur relative $Hr_{(mesure)} = H_1$ ou $Hr_{(mesure)} = Ho_1$ ou $Hr_{(mesure)} = H_2$. Le système peut être configuré pour pouvoir effectuer les trois calculs ou, de préférence ceux de $Hr_{(mesure)} = Ho_1$ et $Hr_{(mesure)} = H_2$ au choix de l'utilisateur ou en fonction de tests logiques.

A partir de la hauteur $Hr_{(mesure)}$ relative de l'eau on peut ensuite calculer une conductivité globale de l'eau soit $EC_{25} = ECmoyen_{(Hmax)}$ soit $EC_{25} = EC_{eau}$. Le système peut être configuré pour pouvoir effectuer les deux calculs au choix de l'utilisateur ou en fonction de tests logiques.

Concernant le calcul de la salinité S, il est possible de calculer la salinité d'une eau à partir de sa conductivité globale. L'institut IFREMER a publié en 2004 (Réf. : Aminot A., Kérouel R., 2004. Hydrologie des écosystèmes marins : paramètres et analyses. Ed Ifremer, 336p. ISBN 2-84433-133-5 ) une formule comportant de très nombreux paramètres et permettant de déterminer la salinité S de manière précise en fonction de la conductivité et de la température.

Cette formule de calcul est :

$S = a0 + (a1 * Rt^{0,5}) + (a2 * Rt) + (a3 * Rt^{1,5}) + (a4 * Rt^2) + (a5 * Rt^{2,5}) + ((T - 15)/(1 + k(T - 15))) * (b0 + (b1 * Rt^{0,5}) + (b2 * Rt) + (b3 * Rt^{1,5}) + (b4 * Rt^2) + (b5 * Rt^{2,5}))$

avec :

$$Rt = (EC_{25}/42,914) / (c0 + (c1 * T) + (c2 + T^2) + (c3 * T^3) + (c4 * T^4))$$

et

| | | |
|---|---|---|
| a0 = 0,0080 | b0 = 0,0005 | c0 = 0,6766097 |
| a1 = -0,1692 | b1 = -0,0056 | c1 = 0,0200564 |
| a2 = 25,3851 | b2 = -0,0066 | c2 = 0,000110426 |
| a3 = 14,0941 | b3 = -0,0375 | c3 = -6,9698E-07 |
| a4 = -7,0261 | b4 = 0,0636 | c4 = 1,0031E-09 |
| a5 = 2,7081 | b5 = -0,0144 | k = 0,0162 |

où $EC_{25}$ est la conductivité globale en mS/cm et $T$ est la température en °C.

Ce calcul pouvant être exécuté par le microcontrôleur est utile dans certaines applications en milieu côtier où il est fréquent que les eaux parasites soient en partie constituées d'infiltrations d'eau de mer.

A noter qu'il est avantageux, plutôt que d'effectuer le calcul complet, de mettre en œuvre dans le système une table de valeurs de salinité précalculées et qui comporte toutes les valeurs de salinité pour des conductivités globales comprises entre 0 et 100 mS/cm et des températures comprises entre 0 et 35°C. Grâce à cela, le temps de calcul de la salinité se résume au temps de parcours de la table.

Il est maintenant décrit le processus d'étalonnage du système qui permet d'obtenir les valeurs des constantes de cellules $K_i$ et, de préférence, aussi la valeur de décalage Z permettant d'éliminer dans les résultats l'effet des courants de fuite pouvant exister au sein du système. La valeur de décalage Z du courant est donc globale et est utilisée pour l'ensemble des paires d'électrodes.

L'étalonnage du système est réalisé par deux opérations :

- Calcul des constantes de cellules $K_i$ ,
- Calcul de des valeurs de décalage, i.e. décalage causé par les courants de fuite. De préférence, on ne met en œuvre qu'une seule valeur de décalage Z par fréquence de mesure pour tout le système.

Ces deux étapes sont réalisées par une même fonction du programme mais de manières indépendantes. En effet, les constantes de cellules $K_i$ sont obtenues en utilisant une solution étalon de conductance connue à une température déterminée, de préférence à 25°C, ou, avantageusement, en effectuant une compensation des effets de la température ramenée à 25°C (voir ci-après), et les valeurs de décalage Z sont obtenues en mettant le capteur hors d'eau, i.e. dans l'air libre et mis au sec.

La mise en œuvre de moyens de communication Modbus est avantageusement utilisé en ce qu'un même registre de commande/d'action est utilisé pour les deux opérations d'étalonnage, la valeur envoyée dans le registre concerné permettant la sélection de l'opération à effectuer.

Ainsi, si cette valeur envoyée est nulle alors c'est le calcul de la données de mise à zéro, Z , du courant de fuite qui sera effectué. Si cette valeur envoyée est supérieure à zéro alors c'est le calcul des constantes de cellules $K_i$ qui sera effectué, la valeur non-nulle envoyée étant la valeur de conductivité de la solution étalon.

Lors des opérations d'étalonnage utilisant la conductivité, des mesures de conductivités locales sont effectuées avec la solution étalon de la même manière que lors d'une mesure pour de l'eau du réseau d'assainissement mais pour la détermination des constantes de cellules $K_i$ aucune correction n'est effectuée lors de la mesure, i.e. $K_i$=1 pour $i = 0, ... N$-1, et ce sont donc des mesures brutes de conductance locales qui sont utilisées pour déterminer $K_i$.

- Pour le calcul de chaque constante de cellule, notée $K_i$, pour chaque paire d'électrode indicée $i$, on utilise chaque valeur de conductance brute (i.e. sans correction, i.e. $K_i$=1 lors de la mesure) mesurée, notée $Cei$, sur la solution étalon et on utilise la formule de calcul de la constante de cellule $K_i$ qui suit :

$$K_i = \frac{\text{ConductivitéEtalon}}{Cei}$$

où ConductivitéEtalon est la conductivité de la solution étalon. A noter que $Cei$ est avantageusement compensée en température à 25°C et on peut donc réaliser l'étalonnage à n'importe quelle température.

De préférence, cet étalonnage est effectué à la température de référence de 25°C ou alors on calcule les conductivités locales avec correction des effets de la température.

A noter que les valeurs de constantes de cellules pouvant être influencées par la fréquence d'excitation des électrodes, il est préférable d'utiliser une fréquence d'excitation adaptée à la plage de valeurs de conductivités que l'on souhaite pouvoir mesurer. Il en est de même pour la mesure de la conductivité de la solution étalon. La liste suivante donne un exemple des relations entre les plages de conductivités mesurables ($\mu$S/cm), les fréquences d'excitation définies préférablement utilisées (Hz) et les conductivités ($\mu$S/cm) conseillées des solutions étalons à utiliser pour la plage de mesure correspondante prévue.

| Plage ($\mu$S/cm) | Fréquence (Hz) | Conductivité de l'étalon ($\mu$S/cm) |
|---|---|---|
| 0 à 1 999 | $50_0$ | 1 413 |
| 2 000 à 9 999 | 1 000 | 5 000 |
| 10 000 et plus | 5 000 | 12 880 |

Selon le cas d'usage, l'utilisateur peut choisir d'étalonner 1, 2 ou 3 plages de mesures. Les valeurs conseillées de conductivité pour les solutions étalons sont des valeurs standards facilement trouvables dans le commerce spécialisé. Le capteur doit être entièrement immergé dans la solution étalon pendant l'opération d'étalonnage des constantes de cellules $K_i$. On détermine donc plusieurs jeux de constantes de cellules, un jeu pour chacune des fréquences d'excitation possibles.

- Pour la correction des courants de fuite on met de préférence en œuvre qu'une seule valeur de décalage Z pour corriger le courant mesuré. La valeurs de décalage Z, destinée à corriger ultérieurement les mesures de courant dans la phase de mesure correspond au courant mesuré alors que le capteur est au sec et à l'air libre et aucune électrode d'excitation n'est sélectionnée.

On peut mettre en œuvre le système de la manière suivante pour obtenir la/les données de mise à zéro Z : On applique par une commande de pont la première fréquence d'excitation, i.e. 500Hz, au pont-en-H 23.

On alimente/active le circuit générateur de tension constante 15 pour générer une tension de 3 V continu.

Alors que le démultiplexeur 26 ne sélectionne aucune électrode d'excitation (e.g. port d'entrée flottant), on acquière des mesures du courant fournies par le circuit de mesure de courant d'alimentation de pont 16, 17, 18, 20, et on mémorise ces mesures de courant acquises.

On coupe l'alimentation/désactive du/le circuit générateur de tension constante 15. On moyenne les mesures de courant et on mémorise le résultat qui est Z.

Ces opérations sont de préférence répétées pour les trois fréquences d'excitation définies d'excitation possibles, i.e. 500Hz, 1000Hz et 5000Hz).

Ainsi, on ne mesure et n'acquière que le courant et on mémorise ce courant qui servira à corriger les mesures de courant ultérieures (hors étalonnage) lors de la phase de mesure.

On peut noter que cette mise en œuvre donne une indication des courant de fuite concernant essentiellement le pont-en-H et fourni une donnée de mise à zéro Z unique (i.e. indépendant des électrodes) pour le courant.

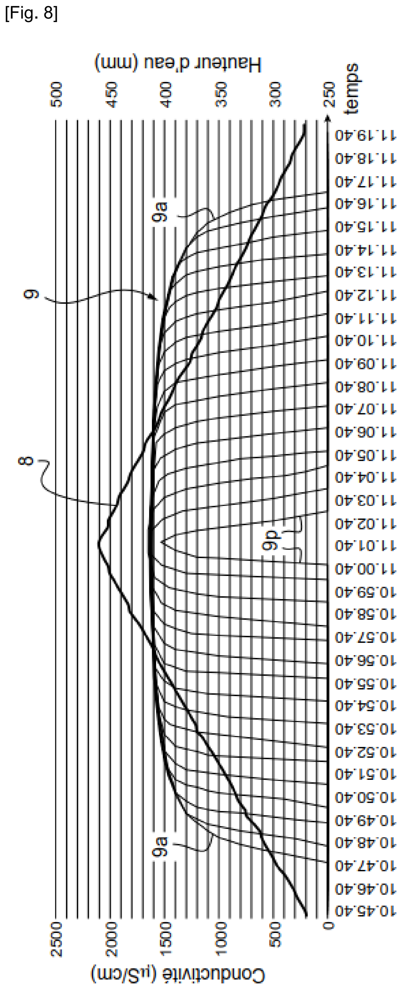

Des exemple de résultats de mesures sont maintenant présenté en relation avec les figures 8 et 9.

Pour la figure 8, le système avec un capteur sans défaut et sans encrassement à 16 électrodes d'excitation a été utilisé et placé dans un bac contenant de l'eau dont le niveau a d'abord été augmenté puis diminué. Les mesures de conductivité 9 pour chacune des 16 électrodes d'excitation sont représentées au cours du temps (représentation conductivité et niveau d'eau versus temps). Sur la figure 8, la conductivité pour l'électrode d'excitation la plus basse du capteur est référencée 9a et la conductivité pour l'électrode d'excitation la plus haute référencée 9p. Le niveau d'eau absolu calculé est représenté par la courbe référencée 8.

Il existe aussi des cas où le capteur peut avoir une ou plusieurs paires d'électrodes défaillantes (c-à-d produisant des mesures de conductivités locales nulles alors qu'elles sont immergées dans l'eau). Il existe aussi des cas où le capteur peut avoir une ou plusieurs paires d'électrodes défectueuses (c-à-d produisant des mesures de conductivités locales non nulles mais très différentes des autres paires d'électrodes) car étant soumises à un milieu comportant des éléments perturbateurs conduisant à des anomalies de mesures (e.g. encrassement).

Plus généralement, une paire d'électrodes est considérée défectueuse si son fonctionnement est altéré. Ainsi, lorsqu'une ou toutes les électrodes d'une paire d'électrodes défectueuses se trouvent sous la surface de l'eau, sa valeur de conductivité locale peut être plus élevée ou plus faible que pour les autres paires d'électrodes immergées. Lorsqu'une ou toutes les électrodes d'une paire d'électrodes défectueuse se trouve au-dessus de la surface de l'eau, sa valeur de conductivité locale peut être non nulle. On met donc avantageusement en œuvre des tests pour comparer les conductivités locales, ceci en fonction de la hauteur relative $Hr_{(mesure)}$ d'eau déterminée pour savoir si les paires d'électrodes sont immergées ou non, afin de rechercher des paires d'électrodes défectueuses. Toutefois, lorsque la différence de conductivité atteint un seuil préalablement défini pour un nombre non nul de paires d'électrodes successives en partant de la paire d'électrodes la plus basse, les paires d'électrodes concernées sont considérées comme se trouvant sous/au contact d'une couche de sédiments et non comme défectueuses.

Les raisons de l'apparition d'une paire d'électrodes défectueuse peuvent être la présence d'un élément perturbateur à la surface des électrodes, la présence d'un dépôt bactérien (biofilm) à la surface des électrodes, la présence d'un dépôt gras à la surface des électrodes, etc. Ces raisons sont donc principalement d'origine externes au capteur.

La figure 9 permet de visualiser certaines anomalies, e.g. une ou plusieurs électrodes défectueuses, sur les mesures de conductivité. Les mesures de conductivité locale 9 pour une partie des électrodes d'excitation du capteur sont représentées au cours du temps pendant lequel le niveau de l'eau monte (représentation conductivité versus niveau d'eau).

Il existe deux types distincts d'encrassements pour des électrodes défectueuses:

- l'encrassement isolant, i.e. entravant le passage du courant, et
- l'encrassement conducteur, i.e. facilitant le passage du courant.

Sur la figure 9, une des électrodes d'excitation (courbe 9x) a été encrassée avec un matériau isolant et une autre (courbe 9y) a été encrassée avec un matériau conducteur (lingette humide).

Les deux types d'encrassement entrainent des réponses très différentes :

- dans le cas du matériau isolant, la réponse pour l'électrode d'excitation (courbe 9x) est simplement atténuée de la même manière que le ferait un dépôt sédimentaire.
- dans le cas du matériau conducteur, la réponse pour l'électrodes d'excitation (courbe 9y) montre une légère conductivité lorsqu'elle est hors de l'eau.

Dans ce dernier cas, si l'encrassement est moins conducteur que l'eau alors la conductivité mesurée lorsque l'électrode d'excitation est totalement immergée sera plus faible que la normale (c'est le cas pour 9y sur la figure 9). A l'inverse, si l'encrassement est plus conducteur que l'eau, alors la conductivité mesurée lorsque l'électrode d'excitation est totalement immergée sera plus forte que la normale.

Le système est configuré pour détecter les différentes formes d'encrassements. La détection d'encrassement pour un encrassement de type isolant se déroule de manière similaire à la détection de dépôts sédimentaires décrite plus haut à ceci près que le seuil de détection est plus faible. Par exemple, si la mesure de conductivité pour l'électrode d'excitation d'indice $i$ donne une valeur de conductivité de x pourcent plus faible que l'électrode d'excitation d'indice $i$+1 (i.e. l'électrode d'excitation $i$+1 juste au-dessus de la précédente i), alors on considère que l'électrode d'excitation d'indice $i$ (donc la parie d'électrodes d'indice $i$) est encrassée. La valeur du seuil de détection x peut être choisie par l'utilisateur et sa valeur par défaut est 10%.

La détection d'encrassement pour un encrassement de type conducteur est réalisée de la manière suivante (pour rappel l'électrode d'excitation de numéro = 0 est l'électrode la plus basse du capteur). Par exemple, si la mesure de conductivité pour l'électrode d'excitation numéro i donne une valeur de conductivité non nulle alors que la mesure pour l'électrode d'excitation numéro i-1 donne une valeur de conductivité nulle, on considère que l'électrode numéro i est encrassée.

Il est maintenant décrit la détection et la correction des anomalies dans les mesures. Dans le cas où une ou quelques paires d'électrodes sont défaillantes (i.e. conductivité nulle alors qu'elle est immergée au contact de l'eau), il est possible de compenser la perte d'information par calcul. A cette fin, il faut détecter les paires d'électrodes dont les valeurs de conductivités locales mesurées sont nulles, i.e. présentant une conductivité de $0\mu$S/cm alors qu'elles sont immergées.

A cette fin on met en œuvre une détection de paires d'électrodes défaillantes, une paire d'électrodes étant défaillante si la conductivité locale pour ladite paire d'électrodes est nulle, c-à-d 0 $\mu$S/cm, alors qu'elle est immergée au contact de l'eau, c-à-d qu'il existe au moins une paire d'électrodes qui donne une conductivité locale non nulle et qui est située à une hauteur supérieure à celle de la paire d'électrodes défaillante.

Avantageusement, le système est configuré pour arrêter les mesures dès qu'un nombre déterminé supérieur ou égal à 2 de paires d'électrodes successives donnent une mesure de conductivité locale de $0\mu$S/cm et/ou dès que deux paires d'électrodes ont été détectées défaillantes. La présence d'une mesure de conductivité nulle pour une électrode d'excitation spécifique au milieu d'autres donnant des mesures non-nulles signale une défaillance de la paire d'électrodes correspondante. Dans ce cas, si le calcul de niveau de l'eau utilise les moyennes des mesures de conductivité locales il se produit alors un décalage dans la courbe d'évolution du niveau de l'eau qu'il est possible de compenser en connaissant le pas d'espacement e entre deux électrodes d'excitation successives le long de la hauteur du capteur (toutefois il persistera une incertitude concernant l'information de niveau autour de l'électrode d'excitation détectée défaillante)

On comprend que les moments d'activations et désactivations et de commandes/contrôles des éléments de l'ensemble électronique peuvent être modifiés par rapport aux moments décrits mais qu'il est préférable que ces moments soient choisis pour que les paires d'électrodes soient le moins longtemps soumises à des polarisations ou au moins qu'elles s'annulent en moyenne (même durée d'application du courant dans un sens et dans l'autre).

Dans certains modes de réalisation dans lesquels les paires d'électrodes ne sont pas alignées verticalement et/ou qu'elles sont sur un capteur flexible épousant une forme non plane, le système peut être configurée pour calculer une hauteur d'eau qui prend en compte la disposition des paires d'électrodes. Ainsi, on envisage un capteur souple épousant la forme circulaire d'une canalisation et on appliquera des calculs pour donner un résultat de hauteur d'eau rapporté sur un axe vertical.

**Revendications**

1. Procédé de mesure d'au moins un paramètre d'une eau d'un réseau d'assainissement, dans lequel on met en œuvre un capteur (1) destiné à être disposé au contact de ladite eau et comportant en surface des électrodes, dont une électrode de référence (11) et un nombre déterminé $N$, supérieur à un, d'électrodes d'excitation (10), l'électrode de référence (11) étant allongée verticalement et les électrodes d'excitation (10) étant étagées d'une manière équidistante entre elles et alignées verticalement parallèlement à l'électrode de référence, chaque électrode d'excitation (10) définissant avec l'électrode de référence (11) une paire d'électrodes, et

   dans une phase de mesures,
   on mesure pour chaque paire d'électrodes du capteur un courant électrique local $I_i$ circulant et un voltage local $V_i$ appliqué, et
   on corrige les courants électriques locaux en ôtant une valeur de décalage $Z$ de mesure de courant pour produire des courants électriques locaux corrigés, et
   on calcule des conductances locales $Co_i$ corrigées en utilisant les courants électriques locaux corrigés, et
   on calcule des conductivités locales $EC_{25i}$ en utilisant les produits des conductances locales $Co_i$ par des constantes de cellule $K_i$ et en utilisant une mesure de température T de ladite eau pour corriger un effet de la température sur les conductances locales, et dans lequel on détermine une hauteur relative $Hr_{(mesure)}$ d'eau en utilisant les conductivités locales $EC_{25i}$, et
   dans lequel on calcule une conductivité globale $EC_{25}$ de ladite eau par une fonction ayant au moins un paramètre qui est la hauteur relative $Hr_{(mesure)}$ déterminée de ladite eau.

2. Procédé selon la revendication 1, dans lequel on calcule la hauteur relative $Hr_{(mesure)} = H_2$ d'eau par:

$$H_2 = (I_{last} * e) + ax^3 + bx^2 + cx + d$$

   Avec :

$$x = \frac{\frac{EC_{last}}{EC_{last-1}} + \frac{EC_{last-1}}{EC_{last-2}}}{2} ,$$

   avec

   $a$, $b$, $c$, $d$ des constantes prédéterminées,
   $e$ le pas d'espacement entre deux électrodes d'excitation successives le long de la hauteur du capteur,
   $EC_{last}$ la conductivité locale $EC_{25i}$ de la dernière paire d'électrodes vers le haut encore en contact avec l'eau, c-à-d la paire d'électrodes de conductivité locale non nulle la plus haute,
   $I_{last}$ est le numéro d'indice de la dernière paire d'électrodes vers le haut encore en contact avec l'eau, la paire d'électrodes la plus basse du capteur ayant un indice égal à zéro.

3. Procédé selon la revendication 1, dans lequel on calcule la hauteur relative $Hr_{(mesure)} = H_1$ d'eau par :

$$H_1 = (EC_{mean} * H_{max} / EC_{max})$$

   avec :

   $EC_{max}$ la conductivité locale $EC_{25i}$ la plus grande parmi les conductivités locales $EC_{25i}$ de l'ensemble des paires d'électrodes du capteur et
   $EC_{mean}$ la conductivité moyenne mesurée sur toutes les paires d'électrodes du capteur, c-à-d la somme des conductivités locales $EC_{25i}$ divisée par le nombre total de paires d'électrodes du capteur, et

$H_{max}$ la hauteur relative maximale d'eau mesurable par le capteur.

4. Procédé selon l'une quelconque des revendications 1 à 3, dans lequel on met en œuvre une détection de paires d'électrodes défaillantes, une paire d'électrodes étant défaillante si la conductivité locale $EC_{25i}$ pour ladite paire d'électrodes est nulle alors qu'elle est surmontée par au moins une paire d'électrodes à conductivité locale non nulle, et on détermine le nombre $m$ de paires d'électrodes défaillantes.

5. Procédé selon la revendication 4, dans lequel on calcule la hauteur relative $Hr_{(mesure)} = Ho_1$ d'eau par :

$$Ho_1 = (EC_{mean} * H_{max} / EC_{max}) + H_{corr}$$

avec :

$EC_{max}$ la conductivité locale $EC_{25i}$ la plus grande parmi les conductivités locales $EC_{25i}$ de l'ensemble des paires d'électrodes du capteur et
$EC_{mean}$ la conductivité moyenne mesurée sur toutes les paires d'électrodes du capteur dans la phase de mesures, c-à-d la somme des conductivités locales $EC_{25i}$ divisée par le nombre total de paires d'électrodes du capteur, et
$H_{max}$ la hauteur relative maximale d'eau mesurable par le capteur, et
$H_{corr}$ une valeur de correction de hauteur calculée par $H_{corr} = e * m$ où $m$ est le nombre de paires d'électrodes défaillantes et où e est le pas d'espacement entre deux électrodes d'excitation successives le long de la hauteur du capteur.

6. Procédé selon l'une quelconque des revendications 2 à 5, dans lequel on calcule la hauteur relative d'eau par $Hr_{(mesure)} = H_1$ ou par $Hr_{(mesure)} = Ho_1$ ou par $Hr_{(mesure)} = H_2$ en fonction d'un résultat de tests logiques où

si ( ($I_{last} < 2$) ou ( ($I_{last} < SEUIL_{capteur}$) et ( (si la paire d'électrodes d'indice $I_{last}$ est non défaillante et si la paire d'électrodes d'indice $I_{last}$ -1 est défaillante) ou (si la paire d'électrodes d'indice $I_{last}$ - 1 est non défaillante et si la paire d'électrodes d'indice $I_{last}$ - 2 est défaillante) ) ) ) alors la hauteur relative d'eau est calculée avec $Hr_{(mesure)} = H_1$ ou, de préférence, avec $Hr_{(mesure)} = Ho_1$ ,
sinon la hauteur relative d'eau est calculée avec $Hr_{(mesure)} = H_2$ ,
$I_{last}$ étant La valeur d'indice de la dernière paire d'électrodes vers le haut encore en contact avec le l'eau, la paire d'électrodes la plus basse du capteur ayant une valeur d'indice égale à zéro, et
$SEUIL_{capteur}$ étant une constante fonction du nombre $N$ d'électrodes d'excitation du capteur.

7. Procédé selon l'une quelconque des revendications 1 à 6, dans lequel l'eau est présente sur un fond nu du réseau d'assainissement et on calcule une hauteur absolue $Ha_{(mesure)}$ d'eau dans le réseau d'assainissement par $Ha_{(mesure)} = Hr_{(mesure)} + H_{offset}$ avec :
$H_{offset}$ la hauteur entre le fond nu du réseau d'assainissement et le bas de l'électrode d'excitation de la paire d'électrode la plus basse du capteur.

8. Procédé selon l'une quelconque des revendications 1 à 7, dans lequel on calcule la conductivité globale $EC_{25}$ par $EC_{25} = EC_{eau}$ avec

$$EC_{eau} = EC_{max} / \propto$$

où

$EC_{max}$ est la conductivité locale la plus grande parmi les conductivités locales de l'ensemble des paires d'électrodes du capteur et
$\propto$ qui est un coefficient de corrélation fonction de la hauteur relative $Hr_{(mesure)}$ d'eau et ayant une valeur comprise entre 0 et 1, $\propto$ étant calculé par :

$$\propto = a_1 * \ln(b1 * Hr_{(mesure)}) + c_1 * Hr_{(mesure)}^3 + d_1 * Hr_{(mesure)}^2 + e_1 * Hr_{(mesure)} + f_1$$

avec

$a_1$, $b_1$, $c_1$, $d_1$, $e_1$ et $f_1$ qui sont des constantes prédéterminées.

9. Procédé selon l'une quelconque des revendications 1 à 8, dans lequel on calcule une salinité S de l'eau en fonction de la température T de l'eau et de la conductivité globale $EC_{25}$.

10. Procédé selon l'une quelconque des revendications 1 à 9, dans lequel on met en œuvre une détection de paires d'électrodes défectueuses, une paire d'électrodes d'indice $i$ étant défectueuse si sa valeur de conductivité locale $EC_{25i}$ est non nulle et en dehors d'une gamme de valeurs définie par les deux bornes $EC_{25i+1}$ * (1 - $Ps$) et $EC_{i+1}$ *(1+ $Ps$) et si $EC_{25i+1}$ est non nulle où Ps est un facteur de sensibilité choisi entre 0,1 et 0,9.

11. Procédé selon l'une quelconque des revendications 1 à 10, dans lequel on met en œuvre une détection de sédiments, une paire d'électrodes d'indice $i$ étant soumise à des sédiments si sa valeur de conductivité locale $EC_{25i}$ est non nulle et est soit inférieure à $EC_{25i+1}$ * (1 - $Pk$), soit supérieure à $EC_{i+1}$ *(1 + $Pk$) où $Pk$ est un facteur de détection choisi entre 0,1 et 0,9, et

dans lequel on calcule une hauteur relative de sédiments $Hr_{sediments}$ par le produit de la valeur d'indice $i$ plus 1 par e qui est le pas d'espacement entre deux électrodes d'excitation successives le long de la hauteur du capteur, soit $Hr_{sediments}$ = e * (i +1).

12. Procédé selon l'une quelconque des revendications 1 à 11, dans lequel, lors de la mesure du courant électrique local $I_i$ et du voltage local $V_i$ on applique à la paire d'électrodes un voltage alternatif périodique de fréquence d'excitation déterminée, ledit voltage alternatif étant en forme de créneaux comportant des plateaux de voltages successifs de signes opposés.

13. Système de mesure d'au moins un paramètre d'une eau d'un réseau d'assainissement comportant un pont-en-H et des moyens matériels dont de calculs, en particulier un microcontrôleur à programme de contrôle spécialement configuré pour assurer la mise en œuvre du procédé de l'une quelconque des revendications 1 à 12.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

Fig. 13

DEBUT

Mesurer la température → Température (°C)

Récupérer les donnéés d'étalonnage en EEPROM → Données d'étalonnage

Num, Electrode = 0

Electrode

Démultiplexer le signal

Démarrer la génération du signal alternatif carré

Relever les valeurs de l'ADC → Tension d'excitation / Courant d'excitation

Arrêter la génération du signal alternatif carré

Calculer la conductivité et application de l'étalonnage → Conductivité (EC25 en µS/cm)

Num, Electrode + 1 ← NON — Electrode >= 16 ?

OUI

Fin

Registres Modbus

Europäisches
Patentamt

European
Patent Office

Office européen
des brevets

**RAPPORT DE RECHERCHE EUROPEENNE**

Numéro de la demande

**EP 25 21 0404**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | FR 2 701 566 A1 (DIVERREZ JEAN CLAUDE [FR]) 19 août 1994 (1994-08-19) | 13 | INV.<br>C02F1/00 |
| A | * abrégé *<br>* figures *<br>* page 5, ligne 1 - ligne 12 *<br>* page 9, ligne 1 - ligne 15 *<br>* page 11, ligne 35 - ligne 38 *<br>----- | 1-12 | G01F23/00<br>G01F23/24<br>G01F23/80<br>G01N27/06<br>G01N27/07<br>G01N27/08 |
| X,D | DE 26 43 964 A1 (COMMISSARIAT ENERGIE ATOMIQUE) 7 avril 1977 (1977-04-07)<br>* figure 1 *<br>* page 6, 2ième paragraphe *<br>* page 9, ligne 17 - page 10, ligne 23 *<br>----- | 13 | G01N33/18 |
| X,D | US 2010/295565 A1 (DRACK EARLE DAVID [US]) 25 novembre 2010 (2010-11-25)<br>* abrégé *<br>* figures *<br>* alinéas [0013], [0018], [0065], [0066] *<br>----- | 13 | |
| A,D | US 2007/164751 A1 (PARACHINI DAVIDE [IT] ET AL) 19 juillet 2007 (2007-07-19)<br>* alinéas [0067] - [0075] *<br>----- | 1-13 | **DOMAINES TECHNIQUES RECHERCHES (IPC)**<br><br>C02F<br>G01F<br>G01N |
| A,D | US 2011/048126 A1 (ADLER SEBASTIAN [DE] ET AL) 3 mars 2011 (2011-03-03)<br>* abrégé *<br>* figure 1 *<br>* alinéas [0041], [0042] *<br>----- | 1-13 | |
| A,D | US 2013/221986 A1 (RIEGEL HARALD [DE]) 29 août 2013 (2013-08-29)<br>* abrégé *<br>* alinéa [0005] *<br>-----<br>-/-- | 1-13 | |

Le présent rapport a été établi pour toutes les revendications

| Lieu de la recherche | Date d'achèvement de la recherche | Examinateur |
|---|---|---|
| Munich | 21 novembre 2025 | Ruchaud, Nicolas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

page 1 de 2

**RAPPORT DE RECHERCHE EUROPEENNE**

Numéro de la demande

EP 25 21 0404

Europäisches Patentamt
European Patent Office
Office européen des brevets

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | US 2018/252569 A1 (NICHOLS ANDREW [GB]) 6 septembre 2018 (2018-09-06) * abrégé * * figures 2a,2b,2c * * alinéas [0072] - [0082], [0102], [0103] * ----- | 1-13 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 21 novembre 2025 | Ruchaud, Nicolas |

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 25 21 0404

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-11-2025

| Document brevet cité au rapport de recherche | | Date de publication | Membre(s) de la famille de brevet(s) | | Date de publication |
|---|---|---|---|---|---|
| FR 2701566 | A1 | 19-08-1994 | EP | 0683892 A1 | 29-11-1995 |
| | | | FR | 2701566 A1 | 19-08-1994 |
| | | | WO | 9418528 A1 | 18-08-1994 |
| DE 2643964 | A1 | 07-04-1977 | BE | 846187 A | 31-12-1976 |
| | | | CH | 608611 A5 | 15-01-1979 |
| | | | DE | 2643964 A1 | 07-04-1977 |
| | | | ES | 451963 A1 | 16-12-1977 |
| | | | FR | 2326689 A1 | 29-04-1977 |
| | | | IT | 1070552 B | 29-03-1985 |
| US 2010295565 | A1 | 25-11-2010 | BR | PI0906699 A2 | 07-07-2015 |
| | | | EP | 2238440 A2 | 13-10-2010 |
| | | | US | 2010295565 A1 | 25-11-2010 |
| | | | US | 2015033830 A1 | 05-02-2015 |
| | | | WO | 2009089339 A2 | 16-07-2009 |
| US 2007164751 | A1 | 19-07-2007 | CA | 2574389 A1 | 19-07-2007 |
| | | | EP | 1811274 A1 | 25-07-2007 |
| | | | US | 2007164751 A1 | 19-07-2007 |
| US 2011048126 | A1 | 03-03-2011 | DE | 102009038744 A1 | 03-03-2011 |
| | | | EP | 2290335 A1 | 02-03-2011 |
| | | | US | 2011048126 A1 | 03-03-2011 |
| US 2013221986 | A1 | 29-08-2013 | CN | 103430016 A | 04-12-2013 |
| | | | DE | 102010060465 A1 | 10-05-2012 |
| | | | US | 2013221986 A1 | 29-08-2013 |
| | | | WO | 2012062798 A1 | 18-05-2012 |
| US 2018252569 | A1 | 06-09-2018 | AU | 2013303921 A1 | 05-03-2015 |
| | | | CA | 2882036 A1 | 20-02-2014 |
| | | | EP | 2885614 A2 | 24-06-2015 |
| | | | ES | 2884096 T3 | 10-12-2021 |
| | | | US | 2015204708 A1 | 23-07-2015 |
| | | | US | 2018252569 A1 | 06-09-2018 |
| | | | WO | 2014027208 A2 | 20-02-2014 |

EPO FORM P0460

Pour tout renseignement concernant cette annexe : voir Journal Officiel de l'Office européen des brevets, No.12/82

EP 4 733 268 A1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2701566
- DE 2643964 A1
- US 2010295565 A1
- US 2007164751 A1
- US 2011048126 A1
- US 2013221986 A1
- US 2018252569 A1

**Littérature non-brevet citée dans la description**

- **AMINOT A.** ; **KÉROUEL R.** Hydrologie des écosystèmes marins : paramètres et analyses. 2004, 336